# EUROPEAN PATENT SPECIFICATION

(11) **EP 1 358 215 B1**
(45) Date of publication and mention of the grant of the patent: **14.09.2011**
(21) Application number: 01271394.7
(22) Date of filing: 17.12.2001
(51) Int. Cl.: C08B 37/00, C08B 37/10, A61K 31/726

(54) **GLYCOSAMINOGLYCANS DERIVED FROM K5 POLYSACCHARIDE HAVING HIGH ANTITHROMBIN ACTIVITY AND PROCESS FOR THEIR PREPARATION**
VON K5-POLYSACCHARID ABGELEITETE GLYCOSAMINOGLYCANE MIT HOHER ANTHITHROMBINWIRKUNG UND VERFAHREN ZU IHRER HERSTELLUNG
GLYCOSAMINOGLYCANES DERIVES DE POLYSACCHARIDE K5 PRESENTANT UNE FORTE ACTIVITE ANTITHROMBOTIQUE ET LEUR PROCEDE DE PREPARATION

(30) Priority: 18.12.2000 US 738879; 12.09.2001 US 950003
(43) Date of publication of application: 05.11.2003
(73) Proprietor: Glycores 2000 srl, 20155 Milan (IT)
(72) Inventor: Oreste Pasqua, 20155 Milan (IT); Zoppetti Giorgio, 20155 Milan (IT)
(74) Representative: Gislon, Gabriele
(86) International application number: PCT/IB2001/002492
(87) International publication number: WO 2002/050125

(56) References cited:
- WO-A-01/72848
- WO-A-97/43317
- WO-A-98/42754
- US-A- 5 795 875
- NAGGI A ET AL: "Generation of anti-factor Xa active, 3-O-sulfated glucosamine-rich sequences by controlled desulfation of oversulfated heparins" CARBOHYDRATE RESEARCH, ELSEVIER SCIENTIFIC PUBLISHING COMPANY. AMSTERDAM, NL, vol. 336, no. 4, December 2001 (2001-12), pages 283-290, XP004328534 ISSN: 0008-6215
- NAGGI ANNAMARIA ET AL.: "Toward a biotechnological heparin through combined chemical and enzymatic modification of the Escherichia coli K5 polysaccharide" SEMINARS IN THROMBOSIS AND HEMOSTASIS, vol. 27, no. 5, 2001, pages 437-443, XP008004483
- NAGASAWA K ET AL: "SOLVOLYTIC DESULFATION OF GLYCOSAMINOGLYCURONAN SULFATES WITH DIMETHYL SULFOXIDE CONTAINING WATER OR METHANOL" CARBOHYDRATE RESEARCH, AMSTERDAM, NL, vol. 58, no. 1, 1 December 1977 (1977-12-01), pages 47-55, XP002032133

## Description

### BACKGROUND OF THE INVENTION

Glycosaminoglycans, such as heparin, heparan sulphate, dermatan sulphate, chondroitin sulphate and hyaluronic acid, are biopolymers industrially extracted from different animal organs.

In particular heparin, principally obtained by extraction from intestinal pig mucosa or bovine lung, is a mixture of chains consisting of repeating disaccharide units formed by an uronic acid (L-iduronic acid or D-glucuronic acid) and by an amino sugar (glucosamipe), joined by a α-1→4 or β-1→4 bonds. The uronic acid unit my be sulphated in position 2 and the glucosamine unit is N-acetylated or N-sulphated and 6-O sulphated. Moreover, glucosamine can contain a sulphate group in position 3 in an amount of about 0.5%. Heparin is a polydisperse copolymer with a molecular weight ranging from about 3,000 to about 30,000 D.

The natural biosynthesis of heparin in mammalians and the properties of this product have been described by Lindahl et al. 1986 in Lane D. and Lindahl U. (Eds.) "Heparin-Chemical and Biological Properties; Clinical Application", Edward Arnold. London, pages space 159-190; and Lindahl U., Feingold, D.S. and Rodén L. (1986) TIBS, 11, 221-225.

The sequence formed by the pentasaccharide region of linkage for Antithrombin III (ATIII) named active pentasaccharide, that is the structure needed for the high affinity binding of heparin to ATIII, is fundamental for heparin activity. This sequence contains one glucosamine unit sulphated in position 3, that is not present in the other parts of the heparin chain. Beside the activity through ATIII, heparin exerts its anticoagulant and antithrombotic activity through the activation of heparin cofactor II (HCII) and a selective inhibition of thrombin. It is known that the minimum saccharidic sequence necessary for HCII activation is a chain containing at least 24 monosaccharides (Tollefsen D.M., 1990 Seminars in Thrombosis and Haemostasis 16, 66-70).

### DESCRIPTION OF THE PRIOR ART

It is known that the capsular polysaccharide K5 isolated from the strain of Escherichia Coli, described by Varm W.F., Schmidt MA, Jann B., Jann K. (1981) in European Journal of Biochemistry 116, 359-364, shows the same sequence of heparin and heparan sulphate precursor (N-acetylheparosan), namely a mixture of chains constituted by repeating disaccharide glucuronyl β-1 → 4-glucosamine Structures. This compound was chemically modified as described by Lormeau et al. in the US patent n. 5,550,116 end by Casu et al. in Carbohydrate Research. 1994, 263, 271-284 or chemically and enzymatically modified in order to obtain products showing in vitro biological activities in coagulation of the same type of heparin as extracted from animal organs.

The chemical and enzymatic modification of polysaccharide K5 was described for the first time in IT 1230785, wherein the polysaccharide K5 (hereinbelow also simply referred to as "K5") is submitted to (a) a N-deacetylation and a N-sulphation; (b) an enzymatic C5-epimerisation of the glucuronic units; (c) a 2-O and/or 6-O-sulphation; and (d) an optional enzymatic 3-O-sulphation, but this method does not give products having a satisfactory activity in respect of that of heparin as extracted from animal organs, hereinafter referred to as "commercial heparin" or "standard heparin", the latter expression designating the fourth International Standard of heparin.

WO 92/17507 discloses a method for preparing heparin-like products starting from K5 by (a) N-deacetylation and N-sulphation, (b) C5 epimerisation, and (c) O-sulphation, step (c) being optionally followed by a N-resulphation. According to this method, the amount of iduronic acid of the resulting product is low (about 20% of the global content of uronic acids).

WO 96/14425 and US 5,958,899 disclose an improved method for the preparation of beparin-like products having a high iduronic acid content, starting from K5, by (a) N-deacetylation and N-sulphation, (b) epimerisation by a C5 epimerase, and (c) sulphation of at least some free hydroxy groups, step (b) being conducted under controlled conditions. The products obtained according to this method lack a considerable amount of N-sulphate groups, lost during the O-sulphation.

WO 97/43317 and US 6,162,797 disclose derivatives of K5 having high anticoagulant activity which are prepared by submitting K5 to (a) N-deacetylation and N-sulphation, (b) C5 epimerisation, (c) O-oversulphation of the epimerised product, previously transformed in a salt thereof with an organic base, and dialysis, and (d) N-resulphation. The products obtained according to this method exhibit a very high global anticoagulant activity.

WO 98/42754 discloses a method for the preparation of glycosaminoglycans, including derivatives of K5, having high antithrombotic activity, said method, in the case of K5, consisting of (a) N-deacetylation and N-sulphation, (b) epimerisation by C5 epimerase, (c) O-oversulphation, (d) partial solvolytic O-desulphation of a salt of the oversulphated product, (e) N-resulphation, and, optionally, (f) O-resulphation. This optional 6-O-resulphation, restoring complete 6-O-sulphation, was expected to further increase the anti-Xa activity (A. Naggi et al., Seminars in Thrombosis and Haemostasis, 2001, 27/5, 437-443). Actually, the products obtained according to this method have the disadvantage of lacking either O-sulphate groups when the optional O-resulphation step (f) is not performed, or N-sulphate groups, which are lost when step (f) is performed. Thus, the incomplete N- or O-, especially 6-O-sulphation (always below 60%) involves, in the case of C5-epimerised K5 polysaccharide, very low anti-Xa values, thus giving a very low anti-Xa/aPPT ratio in the case of incomplete N-sulphation, or low antithrombin or HCII in the case of incomplete 6-O-sulphation, thus giving low anti-IIa/aPTT and/or HCII/aPTT ratios.

### SUMMARY OF THE INVENTION

We have found new glycosaminoglycans derived from K5 polysaccharide from Escherichia Coli with a molecular weight from 3,000 to 30,000, containing from 25% to 50% by weight of the chains with high affinity for ATIII and with a high anticoagulant and antithrombotic activity.

Said glycosaminoglycans are synthesised through a process which substantially comprises in sequence the following steps: (i) N-deacetylation/N-sulphation of the polysaccharide K5, (ii) partial C-5 epimerisation of the carboxyl group of the glucuronic acid moiety to the corresponding iduronic acid moiety, (iii) oversulphation, (iv) selective O-desulphation, (v) optional selective 6-O-sulphation, and (vi) N-sulphation.

Moreover, it has been found that, by carrying out the O-desulphation of the product obtained at the end of step (iii) for a period of time of from 135 to 165 minutes, new compounds are obtained which show the best antithrombotic activity and a bleeding potential lower than that of any other heparin-like glycosaminoglycan.

It has particularly been found that new glycosaminoglycans having a very high antithrombin activity and a bleeding potential lower than that of heparin may be obtained by a process which sequentially comprises (i) N-deacetylation/N-sulphation of the polysaccharide K5, (ii) partial C-5 epimerisation of the carboxyl group of the glucuronic acid moiety to the corresponding iduronic acid moiety, (iii) oversulphation, (iv) time and temperature controlled, selective O-desulphation, (v) 6-O-sulphataon, (vi) N-sulphation, and also comprises an optional depolymerisation step at the end of one of steps (ii)-(vi). Due to this reactions' sequence, these novel glycosaminoglycans are almost completely N-sulphated and highly 6-O-sulphated, thus being different from those obtained by the previously described methods.

More particularly, it has surprisingly been found that, if in step (iv) of the above process the selective O-desulphation of the product obtained at the end of step (iii) is carried out in a mixture dimethyl sulfoxide/methanol for a period of time of from 135 to 165 minutes at a temperature of 50-70°C, new glycosaminoglycans of heparin-type are obtained, said glycosaminoglycans having an anti-Xa activity at least of the same order of standard heparin and a global anticoagulant activity, expressed for example as aPTT, lower than that of standard heparin, a Heparin Cofactor II (HCII) activity at least as high as that of standard heparin and an anti-IIa (antithrombin) activity much higher than that of standard heparin, said novel glycosaminoglycans also having a reduced bleeding risk in respect of commercial heparin. Furthermore, it has been found that by carrying out step (iv) under the above-illustrated conditions, the biological activity with low bleeding risk of the compound obtained at the end of step (vi) is maintained after depolymerisation, said activity of the depolymerised product being expressed by a very high antithrombin activity, anti-Xa and HCII activities of the same order as that of standard heparin and a global anticoagulant activity lower than that of standard heparin. Thus, by carrying out step (iv) under these controlled conditions, it is possible to overcome the above-mentioned disadvantages of the known processes and to obtain new glycosaminoglycans, having improved and selective antithrombin activity, useful as specific coagulation-controlling and antithrombotic agents.

Hereinbelow, derivatives of polysaccharide K5 are also referred to as "deacetylated K5" for N-deacetylated K5 polysaccharide, "N-sulphate K5" for N-deacetylated, N-sulphatad K5 polysaccharide, "C5-epimensed N-sulphate K5" for C5 epimerised, N-deacetylated, N-sulphated polysaccharide K5, "C5-epimerised N,O-sulphate K5" for C5 epimerised, N-deacetylated, N,O sulphated K5 as obtained at the and of step (vi) above, with or without depolymerisation. Unless otherwise specified, starting K5 and its derivatives are intended in form of their sodium salts.

### BRIEF DESCRIPTION OF THE DRAWINGS

Fig. 1 shows the ¹H-NMR spectrum of the K5 polysaccharide working standard obtained according to Vann W.F. et al. 1981 European Journal of Biochemistry 116, 359-364, repeating the purification till the almost complete disappearance of the peaks in the region of 4.9 to 52 ppm of the ¹H-NMR spectrum.
Fig. 2 shows the ¹H-NMR spectrum of the starting K5 polysaccharide of Example 1.
Fig. 3 shows the ¹H-NMR spectrum of the purified K5 polysaccharide used as starting material in Sample 1 (i).
Fig. 4 shows the ¹³C-NMR spectrum of the N-sulphate K5 polysaccharide obtained in example 1(i).
Fig. 5 shows the ¹H-NMR spectrum of the efficiency of the immobilised C-5 epimerase in Example 1(ii-1).
Fig. 6 shows the ¹H-NMR spectrum of the epimerised product obtained at the end of Example 1(ii).
Fig. 7 shows the ¹³C-NMR spectrum of the oversulphated compound obtained in Example 1 (iii).
Fig. 8 shows the ¹³C-NMR spectrum of the desulphated compound obtained in Example 1 (iv).
Fig. 9 shows the ¹³C-NMR spectrum of the compound obtained in Example 1(vi).
Fig. 10 shows the ¹³C-NMR spectrum of the low molecular weight compound obtained in Example 2.

### DETAILED DESCRIPTION OF THE INVENTION

The present invention concerns a process for the preparation of K5 glycosaminoglycans comprising the steps of (i) N-deacetylation/N-sulphation of the polysaccharide K5, (ii) partial C-5 epimerisation of the carboxyl group of the glucuronic acid moiety to the corresponding iduronic acid moiety, (iii) oversulphation, (iv) selective O-desulphation, (v) optional selective 6-O-sulphation, and (vi) N-sulphation, in which step (iv) comprises treating the oversulphated product obtained at the end of step (iii) with a mixture methanol/dimethyl sulfoxide for a period of time of from 135 to 165 minutes.

Preferably, said period of time is of about 150 minutes

The product of the present invention obtained from step (ii) to step (vi) can be chemically depolymerised as described in WO 82/03627, preferably after step (vi).

According to a preferred embodiment, the treatment of the oversulphated product obtained at the end of step (iii) with a mixture methanol/dimethyl sulfoxide is made for a period of time of about 150 minutes at a temperature of about 60°C.

According to this advantageous method, from the oversulphated products prepared according to steps (i) - (iii) new - glycosaminoglycans are obtained which show the best antithrombotic activity and a bleeding potential lower than that of any other heparin-like glycosaminoglycan.

Particularly interesting K5 glycosaminoglycans are obtained according to this advantageous method if, in addition, the partial epimerisation of step (ii) gives at least 40% of iduronic acid moiety, the oversulphation of step (iii) is carried out in an aprotic solvent at a temperature of 40-60°C for 10-20 hours and step (v) of selective 6-O-sulphation is actually performed.

Thus, it is a further object of the present invention to provide a process for the preparation of novel glycosaminoglycans, which comprises
(i) reacting polysaccharide K5 with a N-deacetylated agent, then treating the N-deacetylated product with a N-sulphating agent;
(ii) submitting the N-sulphate K5 thus obtained to a C5-epimerisation by glucuronosyl C5 epimerase to obtain a C5 epimerised N-sulphate K5 in which the iduronic/glucuronic ratio is from 60/40 to 40/60;
(iii) converting the C5 epimerised N-sulphate K5, having a content of 40 to 60% iduronic acid over the total uronic acids, into a tertiary or quaternary salt thereof, then treating the salt thus obtained with an O-sulphating agent in an aprotic polar solvent at a temperature of 40-60°C for 10-20 hours;
(iv) treating a salt with an organic base of the O-oversulphated product thus obtained with a mixture dimethyl sulfoxide/methanol at 50-70 °C for 135-165 minutes;
(v) treating a salt with an organic base of the partially O-desulphated product thus obtained with an O-sulphating agent at a temperature of 0-5°C;
(vi) treating the product thus obtained with a N-sulphating agent; whatever product obtained at the end of one of steps (ii) to (vi) being optionally submitted to a depolymerisation.

K5 used as starting material may be whatever product as obtained by fermentation of wild or cloned, K5 producing Escherichia coli strains. In particular, one of the above-mentioned K5 may be employed, advantageously one of those illustrated by M. Manzoni et al. Journal Bioactive Compatible Polymers, 1996, 11, 301-311 or in WO 01/02597, preferably previously purified.

Advantageous K5 starting materials have a low molecular weight, particularly with a distribution from about 1,500 to about 15,000, advantageously from about 2,000 to about 9,000 with a mean molecular weight of about 5,000, or a higher molecular weight, particularly with a distribution from about 10,000 to about 50,000, advantageously from about 20,000 to about 40,000 with a mean molecular weight of about 30,000. Preferably, starting K5 has a molecular weight distribution from about 1,500 to about 50,000, with a mean molecular weight of 20,000-25,000. All the molecular weights are expressed in Dalton (D). The molecular weight of K5 and of its hereinbelow described derivatives is intended as calculated by using heparin fractions having a known molecular weight as standards.

Typically, for the preparation of K5 polysaccharide from Escherichia Coli, first a fermentation in flask is performed according to the Italian patent application MI99A001465 (WO 01/02597) and using the following medium:

| | | |
|---|---|---|
| Defatted soy | 2 | g/l |
| K₂HPO₄ | 9.7 | g/l |
| KH₂PO₄ | 2 | g/l |
| MgCl₂ | 0.11 g/l | |
| Sodium citrate | 1 | g/l |
| Ammonium sulphate | 1 | g/l |
| Glucose | 2 | g/l |
| Water | 1,000 | ml |
| pH 7.3 | | |

The medium is sterilised at 120 °C for 20 minutes. Glucose is prepared separately as a solution that is sterilised at 120° C for 30 minutes and sterile added to the medium. The flask is inoculated with a suspension of E.Coli cells Bi 8337/41 (O10:K5:H4) from a slant containing tryptic soy agar and incubated at 37°C for 24 hours under controlled stirring (160 rpm, 6 cm of run). The bacterial growth is measured counting the cells with a microscope. In a further step, a Chemap-Braun fermentor with a volume of 14 litres containing the same medium above is inoculated with the 0.1% of the above flask culture and the fermentation is performed with 1vvm aeration (vvm = air volume for liquid volume for minute), 400 rpm stirring and temperature of 37°C for 18 hours. During the fermentation pH, oxygen, residual glucose, produced K5 polysaccharide and bacterial growth are measured.

At the end of the fermentation the temperature is raised to 80°C for 10 minutes. The cells are separated from the medium by centrifugation at 10,000 rpm and the supernatant is ultrafiltrated through a SS 316 (MST) module equipped with PES membranes with a nominal cut off of 800 and 10,000 D to reduce the volume to 1/5. Then K5 polysaccharide is precipitated adding 4 volumes of acetone at 4°C and left to sediment for one night at 4°C and finally is centrifuged at 10,000 rpm for 20 minutes or filtrated.

Then a deproteinisation using a protease of the type II from Aspergillus Orizae in 0.1M NaCl and 0.15 M ethylenediaminotetracetic acid (EDTA) at pH 8 containing 0.5% sodium dodecyl sulphate (SDS) (10 mg/l of filtrate) at 37°C for 90 minutes is performed. The solution is ultrafiltrated on a SS 316 module with a nominal cut off membrane of 10,000 D with 2 extractions with 1M NaCl and washed with water until the absorbance disappears in the ultrafiltrate. K5 polysaccharide is then precipitated with acetone and a yield of 850 mg / l of fermentor is obtained. The purity of the polysaccharide is measured by uronic acid determination (carbazole method), proton and carbon NMR, UV and protein content. The purity is above 80%.

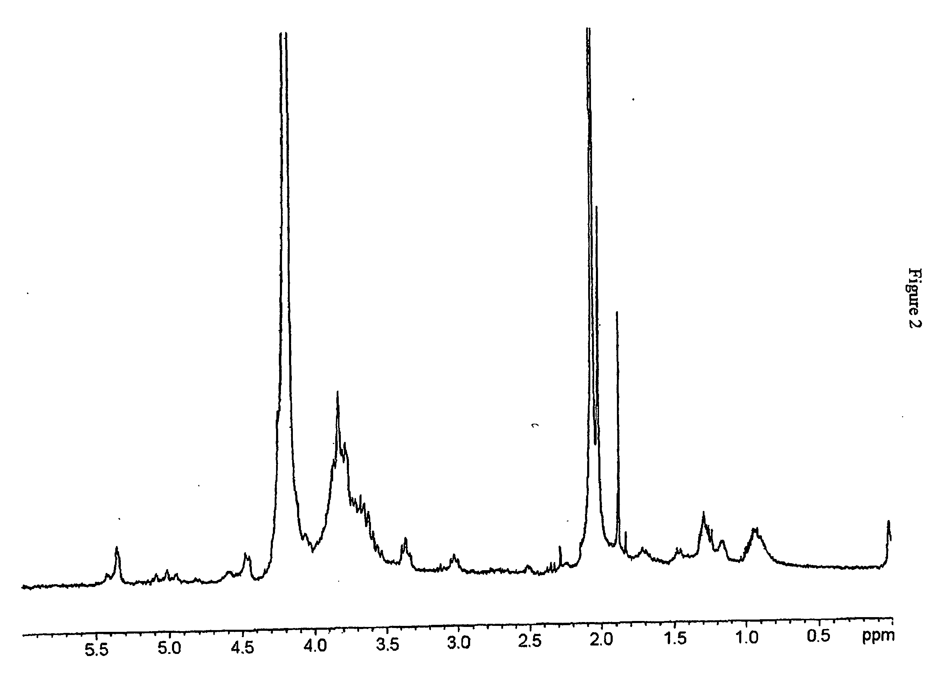

The so obtained polysaccharide is composed of two fractions with different molecular weight, 30,000 and 5,000 D respectively as obtained from the HPLC determination using a 75 HR Pharmacia column and one single fraction with retention time of about 9 minutes using two columns of Bio-sil SEC 250 in series (BioRad) and Na₂SO₄ as mobile phase at room temperature and flow rate of 0.5 ml/minute. The determination is performed against a curve obtained with heparin fractions with known molecular weight. The proton NMR is shown in Fig. 2.

Such a K5 polysaccharide may be used as starting material for the process of the present invention because its purity is sufficient to perform said process. Advantageously, this starting material is previously purified.

A suitable purification of polysaccharide K5 is obtained by treatment with Triton X-100. Typically, Triton X-100 is added to a 1% aqueous solution of the already sufficiently pure, above K5 polysaccharide to a concentration of 5%. The solution is kept at 55°C for 2 hours under stirring. The temperature is raised to 75°C and during the cooling to room temperature two phases are formed. On the upper phase (organic phase) the thermic treatment with the formation of the two phases, is repeated twice. The aqueous phase containing the polysaccharide is finally concentrated under reduced pressure and precipitated with ethanol or acetone. The organic phase is discarded. The purity of the sample is controlled by proton NMR and results to be 95%.

The yield of this treatment is 90%.

In step (i), the starting K5 is submitted to a N-deacetylation and subsequent N-sulphation which are carried out by methods known per se. Typically, 10 g of purified K5 are dissolved in 100-2,000 ml of 2N sodium hydroxide and left to react at 40-80°C for the time necessary to achieve the complete N-deacetylation, which is never above 30 hours. The solution is cooled to room temperature and the pH brought to neutrality with 6N hydrochloric acid.

The solution containing the N-deacetylate K5 is kept at 20-65°C and 10-40 g of sodium carbonate are added together with 10-40 g of a sulphating agent chosen among the available reagents such as the adduct pyridine.sulfur trioxide, trimethylamine.sulfur trioxide and the like. The addition of the sulphating agent is performed during a variable time till 12 hours. At the end of the reaction the solution is brought to room temperature, if necessary and to a pH of 7.5-8 with a 5% solution of hydrochloric acid.

The product is purified from salts by known technologies, for instance by diafiltration using a spiral membrane with 1,000 D cut off (prepscale cartridge - Millipore). The process is finished when the conductivity of the permeate is less than 1,000 µS, preferably less than 100 µS. The volume of the product obtained is concentrated till 10% polysaccharide concentration using the same filtration system as concentrator. If necessary the concentrated solution is dried with the known technologies.

The N/sulphate/N-acetyl ratio ranges from 10/0 to 7/3 measured by carbon 13 NMR.

Step (ii) of C5 epimerisation according to the present invention can be performed with the enzyme glucuronosyl C5 epimerase (also called C5 epimerase) in solution or its immobilised form, in particular to give at least 40% epimerisation, for example in the presence of specific divalent cations, said enzyme being chosen from the group including recombinant glucuronosyl C5 epimerase, glucuronosyl C5 epimerase from murine mastocytoma and glucuronosyl C5 epimerase extracted from bovine liver and said divalent cations being chosen from the group comprising Ba, Ca, Mg and Mn, as described in WO 01/72848. Typically, the epimerisation is carried out as follows.

### - C5 epimerisation with the enzyme in solution.

From 1.2 x 10⁷ to 1.2 x 10¹¹ cpm (counts per minute) of natural or recombinant C5 epimerase, calculated according to the method described by Campbell P. et al. Analytical Biochemistry 131, 146-152 (1983), are dissolved in 2-2,000 ml of 25 mM Hepes buffer at a pH comprised between 5.5 and 7.4, containing 0.001-10 g of N-sulphate K5 and one or more of the ions chosen among barium, calcium, magnesium, manganese at a concentration ranging from 10 and 60 mM. The reaction is performed at a temperature ranging from 30 and 40°C, preferably 37°C for 1-24 hours. At the end of the reaction the enzyme is inactivated at 100°C for 10 minutes.

The product is purified by a passage on a diethylaminoethyl-resin (DEAE-resin) or a DEAE device Sartobind and unbound with 2M NaCl and finally desalted on a Sephadex G-10 resin or it is purified by precipitation with 2 volumes of ethanol and passage on an IR 120 H⁺ resin to make the sodium salt.

The product obtained shows an iduronic acid/glucuronic acid ratio between 40:60 and 60:40 calculated by ¹H-NMR as already described in WO 96/14425.

### - C5 epimerisation with immobilised enzyme.

The enzyme C5 epimerase, natural or recombinant, can be immobilised on different inert supports including resins, membranes or glass beads derivatised with reactive functional groups using the most common technologies of linkage for the enzymes such as cyanogen bromide, glutaraldehyde, carbodiimide or making the enzyme react with a ionic exchange resin or adsorbe on a membrane. According to the present invention the reactions of binding of the enzyme to the inert support are performed in presence of the substrate N-sulphate K5 to avoid the active site of the enzyme to link with loss of activity. The measure of the activity of the immobilised enzyme is performed by recirculating the amount of N-sulphated K5 that theoretically can be epimerised by that amount of cpm of immobilised enzyme onto a column of the immobilised enzyme in presence of 25 mM Hepes, 0.1M KCl, 0.01% Triton X-100 and 0.15 M EDTA pH 7.4 buffer at 37°C overnight at a flow rate of 0.5 ml/minute. After the purification by DEAE chromatographic method and desalting on Sephadex G-10 the product is freeze dried and the content of iduronic acid is calculated by proton NMR.

The ratio iduronic acid/glucuronic acid shall be about 30/70.

A volume of 20-1,000 ml of 25 mM Hepes buffer at a pH between 6 and 7.4 containing one or more ions chosen among barium, calcium, magnesium, manganese at a concentration between 10 and 60 mM and 0.001-10 g N-sulphated K5 kept at a temperature between 30 and 40 °C, are recirculated at a flow rate of 30-160 ml/hour for 1-24 hours in a column containing from 1.2 x 10⁷ to 3 x 10¹¹ cpm equivalents of the enzyme immobilised on the inert support kept at a temperature from 30 to 40°C. At the end of the reaction the sample is purified with the same methods indicated in the epimerisation in solution.

The ratio iduronic acid/glucuronic acid of the product obtained ranges between 40:60 and 60:40.

According to a preferred embodiment, said C5 epimerisation is performed with the enzyme in its immobilized form and comprises recirculating 20-1,000 ml of a solution of 25 mM Hepes at pH of from 6 to 7.4 containing 0.001-10 g of N-sulphated K5 and one of said cations at a concentration between 10 and 60 mM through a column containing from 1.2 x 10⁷ to 3 x 10¹¹cpm of the immobilised enzyme on an inert support, said pH being preferably about 7 and said C5 epimerisation being preferably performed at a temperature of about 30°C by recirculating said solution with a flow rate of about 200 ml/hour for a time of about 24 hours.

Step (iii), consisting of an O-oversulphation, is carried out by previously converting the C5 epimerised N-sulphate K5 into a tertiary or quaternary salt thereof and then by treating said salt with an O-sulphating agent at a temperature of 40-60°C for 10-20 hours. Typically, the solution containing the epimerised product of step (ii) at a concentration of 10% is cooled at 10°C and passed through an IR 120 H⁺ column or equivalent (35-100 ml). Both the column and the container of the product are kept at 10°C. After the passage of the solution the resin is washed with deionised water until the pH of the flow through is more than 6 (about 3 volumes of deionised water). The acidic solution is kept to neutrality with a tertiary or quaternary amine such as tetrabutylammonium hydroxide (15% aqueous solution) obtaining the ammonium salt of the polysaccharide. The solution is concentrated to the minimum volume and freeze-dried. The product obtained is suspended in 20-500 ml of dimethyl formamide (DMF) or dimethyl sulfoxide (DMSO) and added with 15-300 gr. of a sulphating agent such as the adduct pyridine.SO₃ in the solid form or in solution of DMF or DMSO. The solution is kept at 20-70°C for 2-24 hours, preferably at 40 - 60°C for 15-20 hours. At the end of the reaction the solution is cooled to room temperature and added with acetone saturated with sodium chloride till complete precipitation. The precipitate is separated from the solvent by filtration, solubilised into the minimum amount of deionised water (for instance 100 ml) and added with sodium chloride to obtain a 0.2M solution. The solution is brought to pH 7.5-8 with 2N sodium hydroxide and added with acetone till complete precipitation. The precipitate is separated from the solvent by filtration. The solid obtained is dissolved into 100 ml of deionised water and purified from the residual salts by ultrafiltration as described in step (i).

Part of the product obtained is freeze dried for the structural analysis of the oversulphated product by ¹³C-NMR. The content of sulphates per disaccharide of the product obtained is 2.8-3.5 calculated according to Casu B. et al. Carbohydrate Research 1975, 39, 168-176. The position 6 of the glucosamine is sulphated at 80-95% and the position 2 is completely unsulphated. The other sulphate groups are present in position 3 of the amino sugar and in positions 2 and 3 of the uronic acid.

Step (iv), consisting of a selective O-desulphation, is the key step of the process of the present invention, because it allows the preparation, at the end of step (vi), of glycosaminoglycans that, after depolymerisation, give low molecular weight products substantially maintaining a high antithrombin activity. Typically, the solution containing the product of step (iii) is passed through a cationic exchange resin IR 120 H+ or equivalent (35-100 ml). After the passage of the solution, the resin is washed with deionised water till the pH of the flow through is more than 6 (about 3 volumes of deionised water). The acidic solution is brought to neutrality with pyridine. The solution is concentrated to the minimum volume and freeze dried. The product obtained is treated with 20-2,000 ml of a solution of DMSO/methanol (9/1 V/V) and the solution is kept at 50-70°C for 135-165 minutes, preferably at about 60°C for about 150 minutes. Finally the solution is added with 10-200 ml of deionised water and treated with acetone saturated with sodium chloride to complete precipitation.

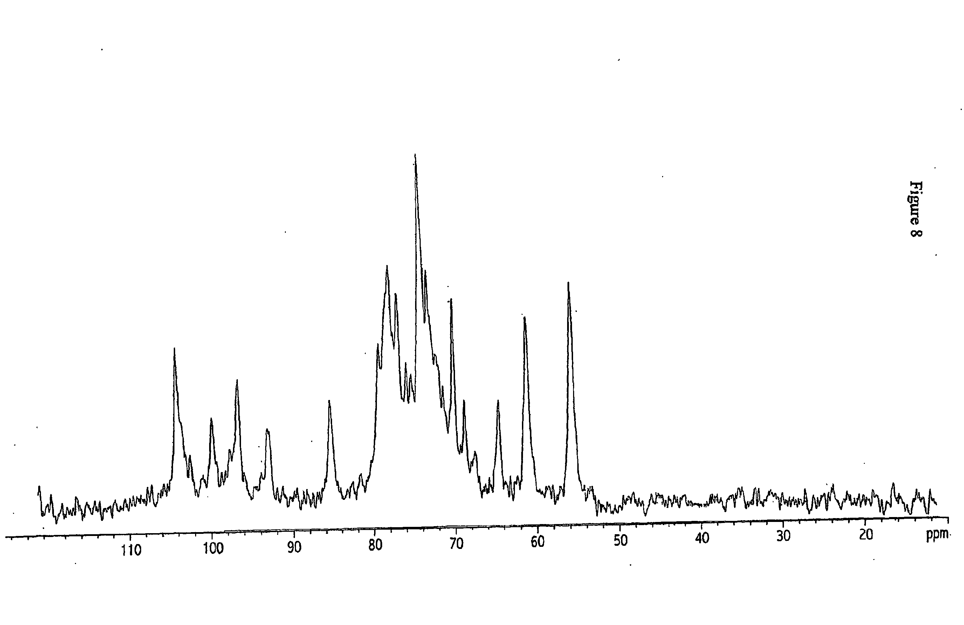

With the selective O-desulphation, the sulphate groups in position 6 of the glucosamine are eliminated first, then the sulphate groups in position 3 and 2 of the uronic acid and finally the sulphate group in position 3 of the amino sugar. The ¹³C-NMR spectrum of the sample obtained (Fig. 8) shows the complete N-desulphation of the glucosamine residue (signal at 56 ppm) and the almost complete 6-O-desulphation with the decreasing of the signal at 67.6 ppm and the appearance of the signal at 62.2 ppm. The signals at 65 and 86 ppm show the 2-O-sulphated iduronic acid and the 3-O-sulphated glucuronic acid, respectively. The solid obtained is purified by diafiltration according to known methods, for instance by using a spiral membrane with 1,000 D cut off (prepscale cartridge - Millipore). The process is finished when the conductivity of the permeate is less than 1,000 µS, preferably less than 100 µS. The volume of the product obtained is concentrated till 10% polysaccharide concentration using the same filtration system as concentrator. If necessary, the concentrated solution is dried by conventional technologies.

Step (v), consisting of a 6-O-sulphation, must also be carried out if, after a depolymerisation step following step (vi) below, compounds having a high antithrombin activity, anti-Xa, HCII activities as high as those of heparin and a low aPTT are desired. The selective 6-O-sulphation is carried out by converting the selectively O-desulphated product into a tertiary or quaternary salt thereof and treating said salt with an O-sulphating agent at low temperature, more particularly at 0-5°C for 0.5-3 hours. Typically, the 6-O-sulphation is carried out as illustrated above for step (iii) of O-sulphation. The solid obtained is purified by diafiltration as described in step (iv). A small amount is freeze-dried for the structural analysis by ¹³C-NMR. If the content of 6-O sulphate groups calculated by NMR, as described by Casu et al. Arzneimittel-Forschung Drug Research, 1983, 33, 135-142, is less than about 85%, step (v) is repeated.

Step (vi) must be performed because a non-negligible percent of N-sulphate groups is lost during the O-oversulphation step. Thus, the solution obtained in step (v) is treated as described in step (i) for the N-sulphation in order to isolate the C5-epimerised N,O-sulphate K5 of the invention.

Whatever high molecular weight product obtained at the end of one of steps (ii) to (vi) may be chemically depolymerised in order to obtain, as final products, low molecular weight glycosaminoglycans having high antithrombin activity, anti-Xa and HCII activities of the same order of those of standard heparin and an aPTT activity lower than that of standard heparin.

Generally, the process of the present invention is performed by carrying out steps (i)-(vi) sequentially and submitting the high molecular weight, C5-epimerised N,O-sulphate K5 obtained at the end of step (vi) to depolymerisation. Of course, such a depolymerisation is not necessary to prepare a low molecular weight C5-epimerised N,O-sulphate K5 if, as starting material, a low molecular weight fraction of K5, optionally previously purified, is used.

The depolymerisation may be carried out according to the known methods for the depolymerisation of heparin, for example by nitrous acid and subsequent reduction with sodium borohydride (WO 82/03627 - EP 37319), by sodium periodate (EP 287477), by free radicals (EP 121067) or by β-elimination (EP 40144), in order to obtain, as final product, a glycosaminoglycan constituted by a mixture of chains in which at least 80% of said chains have a molecular weight distribution ranging from about 2,000 to about 10,000 with a mean molecular weight of from about 4,000 to about 8,000.

The glycosaminoglycans obtained by the process of the invention are characterised by proton and carbon 13 NMR and by biological tests like anti-Xa, aPTT, HCII, Anti-IIa and affinity for ATIII. As already mentioned above, the sulphation degree, namely the number of sulphate groups per disaccharide unit expressed as sulphate/carboxyl ratio (SO₃⁻/COO⁻), is determined as described by Casu et al., Carbohydrate Research, 1975, 39, 168-176.

The product obtained at the end of step (vi), without any depolymerisation, may also be fractionated by chromatography on resin or ultrafiltration to obtain low molecular weight fractions of from 2,000 to 8,000 D and high molecular weight fractions of from 25,000 to 30,000 D.

The novel C5-epimerised N,O-sulphate K5 glycosaminoglycans obtained at the end of the process of the present invention are generally isolated in form of their sodium salt. Said sodium salt may be converted into another salt. Said other salt may be another alkaline metal salt or an alkaline-earth metal, ammonium, (C₁-C₄)trialkylammonium, aluminium or zinc salt.

The products obtained by the process of the present invention show comparable activity to the extractive heparin in the anti-Xa test and reduced global anticoagulant activity (aPTT method) while the values of the tests involving inhibition of thrombin, heparin cofactor II (HCII) and anti-IIa activities, are of the same order as or markedly higher than those of standard heparin. These characteristics of the product obtained are predictive of better coagulation controlling and antithrombotic properties and lower side effects, such as bleeding effect, than those of commercial heparins and of other known anticoagulant glycosaminoglycans.

Thus it is a further object of the present invention to provide novel C5-epimerised N,O-sulphate K5 glycosaminoglycans obtainable by a process which comprises
(i) reacting K5 with a N-deacetylating agent, then treating the N-deacetylated product with a N-sulphating agent;
(ii) submitting the N-sulphate K5 thus obtained to a C5-epimerisation by glucuronosyl C5 epimerase to obtain a C5-epimerised N-sulphate K5 in which the iduronic/glucuronic ratio is from 60/40 to 40/60;
(iii) converting the C5 epimerised N-sulphate K5, having a content of 40 to 60% iduronic acid over the total uronic acids, into a tertiary or quaternary salt thereof, then treating the salt thus obtained with an O-sulphating agent in an aprotic polar solvent at a temperature of 40-60°C for 10-20 hours;
(iv) treating a salt with an organic base of the O-oversulphated product thus obtained with a mixture dimethyl sulfoxide/methanol at 50-70 °C for 135-165 minutes;
(v) treating a salt with an organic base of the partially O-desulphated product thus obtained with an O-sulphating agent at a temperature of 0-5°C;
(vi) treating the product thus obtained with a N-sulphating agent; whatever product obtained at the end of one of steps (ii) to (vi) being optionally submitted to a depolymerisation and the sodium salt of the end product being optionally converted into another salt.

Particularly advantageous C5-epimerised N,O-sulphate K5 glycosaminoglycans are those obtainable by the above process, in which step (iv) is carried out in a 9/1 (V/V) dimethyl sulfoxide/methanol mixture at about 60°C for about 150 minutes.

A preferred class of glycosaminoglycans derived from K5 is obtainable by performing steps (i)-(vi) above on a previously purified K5, whereby step (iv) is carried out by heating at about 60°C in a 9/1 dimethyl sulfoxide/methanol mixture for about 150 minutes, and optionally submitting the C5-epimerised N,O-sulphate K5 thus obtained to a nitrous acid depolymerisation and to a subsequent sodium borohydride reduction.

Advantageously, said other salt is another alkaline metal, an alkaline-earth metal, ammonium, (C1-C4)trialkylammonium, aluminium or zinc salt.

The C5-epimerised N,O-sulphate K5 glycosaminoglycans obtainable according to the process comprising steps (i)-(vi) above, including the optional depolymerisation and salt formation, have the structure I as illustrated hereinbelow.

Thus, it is another object of the present invention to provide novel glycosaminoglycans constituted by a mixture of chains in which at least 90% of said chains has the formula I 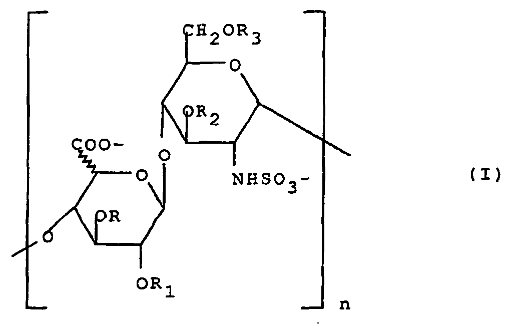 wherein 40-60% of the uronic acid units are those of iduronic acid, n is an integer of from 3 to 100, R, R₁, R₂ and R₃ represent a hydrogen atom or a SO₃⁻ group and from about 65% to about 50% of R, R₁, R₂ and R₃ being hydrogen and the remaining being SO₃⁻ groups distributed as follows
- R₃ is from about 85% to about 95% SO₃⁻;
- R₂ is from about 17 to about 21 % SO₃⁻;
- R₁ is from about 15 to about 35% SO₃⁻ in iduronic units and 0 to 5% SO₃⁻ in glucuronic units;
- R is from about 20 to about 40% SO₃⁻ in glucuronic units and 0 to 5% in iduronic units;
- the sum of the SO₃⁻ percent in R₁, glucuronic units, and in R, iduronic units, is from 3 to 7%;

R₁ and R being not simultaneously SO₃⁻ and being both hydrogen in 25-45% of the uronic acid units; the sulphation degree being from about 2.3 to about 2.9, and the corresponding cation being a chemically or pharmaceutically acceptable one.

In this context, the expression "chemically acceptable" is referred to a cation which is useful for the chemical syntheses, such as ammonium or (C₁-C₄)trialkylammonium ion, or for the purification of the products.

Advantageously, from about 60% to about 55% of R, R₁, R₂ and R₃ are hydrogen and the remaining are SO₃⁻ groups for a sulphation degree of from about 2.4 to about 2.7.

Advantageous low molecular weight glycosaminoglycans are constituted by a mixture of chains in which at least 80% of said chains have the formula I wherein n is from 3 to 15.

Among these low molecular weight glycosaminoglycans, those in which said mixture of chains has a molecular weight distribution ranging from about 2,000 to about 10,000, with a mean molecular weight of from about 4,000 to about 8,000 are particularly advantageous.

Preferred glycosaminoglycans of this class are constituted by a mixture of chains with a mean molecular weight of from about 6,000 to about 8,000, in which at least 90% of said chains have the formula I above, wherein about 55% of the uronic acid units are those of iduronic acid and R₃ is from about 85% to about 90% SO₃⁻; R₂ is about 20% SO₃⁻; R₁ is from about 25% to about 30% SO₃⁻ in iduronic units and 0 to about 5% SO₃⁻ in glucuronic units; R is from about 30% to about 35% SO₃⁻ in glucuronic units and 0 to about 5% in iduronic units; the sum of the SO₃⁻ percent in R₁, glucuronic units and in R, iduronic units, is about 5%; R₁ and R being not simultaneously SO₃⁻ and being both hydrogen in from about 30% to about 40% of the uronic acid units; the sulphation degree being from about 2.5 to about 2.7, the corresponding cation being a chemically or pharmaceutically acceptable one.

A particularly preferred low molecular weight glycosaminoglycan of this class is constituted by a mixture of chains with a mean molecular weight of about 7,000, in which at least 90% of said chains have the formula I above, wherein about 55% of the uronic acid units are those of iduronic acid and
- R₃ is about 85% SO₃⁻;
- R₂ is about 20% SO₃⁻;
- R₁ is about 25% SO₃⁻ in iduronic units and 0 to about 5% SO₃⁻ in glucuronic units;
- R is about 30% SO₃⁻ in glucuronic units and 0 to about 5% in iduronic units;
- the sum of the SO₃⁻ percent in R₁, glucuronic units and in R, iduronic units, is about 5%;

R₁ and R being not simultaneously SO₃⁻ and being both hydrogen in about 40% of the uronic acid units; the sulphation degree being about 2.55, the corresponding cation being a chemically or pharmaceutically acceptable one.

The percent of the sulphate group in the 3-position of the glucuronic acid and 2-position of iduronic acid moieties have been determined by ¹³C-NMR on the compound obtained after step (iv), by measuring the areas of the peaks at 83 and 62 ppm, attributable to the 3-O-sulpho-glucuronic acid unit and, respectively, to the 2-O-sulpho-iduronic acid unit and by considering that the percent of the added SO₃⁻ groups in step (vi), in respect of the total amount of sulphate groups, is negligible.

Advantageous chemically and pharmaceutically acceptable cations are those derived from alkaline metals, alkaline-earth metals, ammonium, (C1-C4)trialkylammonium, aluminium and zinc, sodium and calcium ions being particularly preferred.

Advantageous high molecular weight glycosaminoglycans are constituted by a mixture of chains in which at least 80% of said chains have the structure I wherein n is from 20 to 100.

Among these glycosaminoglycans, those in which said mixture of chains has a molecular weight distribution ranging from about 9,000 to about 60,000, with a mean molecular weight of from about 12,000 to about 30,000 are preferred.

A particularly preferred high molecular weight glycosaminoglycan of this class is constituted by a mixture of chains with a mean molecular weight of 14,000-16,000, in which at least 90% of said chains have the formula I above, wherein about 55% of the uronic acid units are those of iduronic acid and
- R₃ is from about 85% to about 90% SO₃⁻;
- R₂ is about 20% SO₃⁻;
- R₁ is from about 25% to about 30% SO₃⁻ in iduronic units and 0 to about 5% SO₃⁻ in glucuronic units;
- R is from about 30% to about 35% SO₃⁻ in glucuronic units and 0 to about 5% in iduronic units;
- the sum of the SO₃⁻ percent in R₁, glucuronic units and in R, iduronic units, is about 5%;
R₁ and R being not simultaneously SO₃⁻ and being both hydrogen in from about 30% to about 40% of the uronic acid units; the sulphation degree being from about 2.5 to about 2.7, the corresponding cation being a chemically or pharmaceutically acceptable one. A preferred mean molecular weight is about 15,700.

The novel glycosaminoglycans obtainable by the process sequentially comprising steps (i)-(vi) above, including optional depolymerisation and salt formation, in particular those constituted by a mixture of chains in which at least 90% of said chains has the formula I, in which R, R₁, R₂ and R₃ are as defined above and in which the corresponding cation is a chemically or pharmaceutically acceptable one, preferably a sodium or calcium ion, show interesting biological activities on the coagulation parameters. Particularly, said novel glycosaminoglycans exhibit anti-Xa and HCII activities at least of the same order of that of standard heparin, an anti-IIa (antithrombin) activity higher than that of standard heparin activity and a global anticoagulant activity (expressed as aPTT titre) lower than that of standard heparin. More particularly, said novel glycosaminoglycans show ratios anti-Xa/aPTT, HCII/aPTT and anti-IIa/anti-Xa of from 1.5 to 3 and a HCII/antiXa ratio of from 1 to 3.

Due to their characteristics, the glycosaminoglycans of the present invention may be used alone or in combination with acceptable pharmaceutical excipients or diluents, for the control of the coagulation and for the antithrombotic treatment, in particular for the prevention or for the treatment of thrombosis.

Therefore, it is a further object of the present invention to provide pharmaceutical compositions comprising, as an active ingredient, a pharmacologically active amount of a C5-epimerised N,O-sulphate K5 glycosaminoglycan obtainable according to the process wherein steps (i)-(vi) above, including the optional depolymerisation and formation of a pharmaceutically acceptable salt are performed as illustrated above in combination with pharmaceutically acceptable excipients or diluents.

Preferably, the active ingredient is obtainable according to steps (i)-(vi) above, including pharmaceutically acceptable salt formation, starting from a previously purified K5 and carrying out step (iv) in dimethyl sulfoxide/methanol 9/1 (V/V) at about 60°C for about 150 minutes, and submitting the C5-epimerised N,O-sulphate K5 obtained at the end of step (vi) to depolymerisation. Preferably, the thus obtainable C5-epimerised N,O-sulphate K5 glycosaminoglycan active ingredient is in form of an alkaline metal, alkaline-earth metal, aluminium or zinc salt

Particularly, the present invention provides pharmaceutical compositions comprising a pharmacologically effective amount of a glycosaminoglycan constituted by a mixture of chains in which at least 90% of said chains has the formula I above, wherein 40-60% of the uronic acid units are those of iduronic acid, n is an integer of from 3 to 100, R, R₁, R₂ and R₃ represent a hydrogen atom or a SO₃⁻ group and from about 65% to about 50% of R, R₁, R₂ and R₃ being hydrogen and the remaining being SO₃⁻ groups distributed as follows
- R₃ is from about 85% to about 95% SO₃⁻;
- R₂ is from about 17 to about 21% SO₃⁻;
- R₁ is from about 15 to about 35% SO₃⁻ in iduronic units and 0 to 5% SO₃⁻ in glucuronic units;
- R is from about 20 to about 40% SO₃⁻ in glucuronic units and 0 to 5% in iduronic units;
- the sum of the SO₃⁻ percent in R₁, glucuronic units, and in R, iduronic units, is from 3 to 7%;

R₁ and R being not simultaneously SO₃⁻ and being both hydrogen in 25-45% of the uronic acid units; the sulphation degree being from about 2.3 to about 2.9, and the corresponding cation being a pharmaceutically acceptable one, as an active ingredient, and a pharmaceutical carrier.

More particularly the above compositions are indicated for the control of the coagulation or for the prevention or treatment of thrombosis

In said pharmaceutical compositions, for intravenous, subcutaneous or topical use, said glycosaminoglycan active ingredient is present in an effective dose for the prevention or treatment of diseases caused by disorders of the coagulation system, such as arterial or venous thrombosis, for the treatment of haematomas or as coagulation controlling agents during surgical operations.

In preparations for intravenous or subcutaneous use, the glycosaminoglycan active ingredient is dissolved in water, if necessary in the presence of a buffer and the solution is introduced in vials or syringes under sterile conditions.

Unit doses of said pharmaceutical compositions contain from 5 to 100 mg advantageously from 20 to 50 mg of active ingredient dissolved in 0.1 to 2 ml of water.

In compositions for topical use, the glycosaminoglycan active ingredient is mixed with pharmaceutically acceptable carriers or diluents known in the art for the preparation of gels, creams, ointments, lotions or solutions to be sprayed. In said compositions, the glycosaminoglycan active ingredient is present in a concentration of from 0.01% to 15% by weight advantageously.

Advantageous pharmaceutical compositions comprise, as an active ingredient, a pharmacologically active amount of a glycosaminoglycan constituted by a mixture of chains of formula I, as illustrated above, in which the counter-ion is a pharmaceutically acceptable one, advantageously a cation selected from the group consisting of alkaline metal, alkaline-earth metal, aluminium and zinc ions, preferably the sodium or calcium ion, and a pharmaceutical carrier.

Among these advantageous glycosaminoglycans, those which contain at least 80% of chains of formula I wherein n is from 3 to 15 or from 20 to 100 are preferred active ingredients, those in which the mixture of chains has a molecular weight distribution ranging from about 2,000 to about 10,000, with a mean molecular weight of from about 4,000 to about 8,000 or a molecular weight distribution ranging from about 9,000 to about 60,000, with a mean molecular weight of from about 12,000 to about 30,000, being particularly preferred.

Particularly advantageous pharmaceutical compositions comprise, as an active ingredient, a glycosaminoglycan constituted by a mixture of depolymerised chains in which at least 90% of said chains have the formula I above, wherein 40-60% of the uronic acid units are those of iduronic acid, n is an integer of from 3 to 100, R, R₁, R₂ and R₃ represent a hydrogen atom or a SO₃⁻ group, from about 65% to about 50% of R, R₁, R₂ and R₃ being hydrogen and the remaining being SO₃⁻ groups distributed as follows
- R₃ is from about 85% to about 95%, preferably about 85%, SO₃⁻;
- R₂ is from about 17 to about 21%, preferably about 20%, SO₃⁻;
- R₁ is from about 15 to about 35%, preferably about 25%, SO₃⁻ in iduronic units and 0 to about 5% SO₃⁻ in glucuronic units;
- R is from about 20 to about 40% SO₃⁻ in glucuronic units and 0 to about 5% in iduronic units;
- the sum of the SO₃⁻ percent in R₁, glucuronic units, and in R, iduronic units, is from about 3 to about 7%;

R₁ and R being not simultaneously SO₃⁻ and being both hydrogen in 25-45% of the uronic acid units; the sulphation degree being from about 2.3 to about 2.9, preferably from about 2.4 to about 2.7, and the corresponding cation being a pharmaceutically acceptable one, said mixture of depolymerised chains containing at least 80% of said chains with a molecular weight distribution in the range of from about 2,000 to about 10,000 and a mean molecular weight of from about 4,000 to about 8,000.

Advantageous pharmaceutical compositions comprise, as an active ingredient, a pharmacologically active amount of a glycosaminoglycan constituted by a mixture of chains with a mean molecular weight of from about 6,000 to about 8,000, in which at least 90% of said chains have the formula I above, wherein about 55% of the uronic acid units are those of iduronic acid and R₃ is from about 85% to about 90% SO₃⁻; R₂ is about 20% SO₃⁻; R₁ is from about 25% to about 30% SO₃⁻ in iduronic units and 0 to about 5% SO₃⁻ in glucuronic units; R is from about 30% to about 35% SO₃⁻ in glucuronic units and 0 to about 5% in iduronic units; the sum of the SO₃⁻ percent in R₁, glucuronic units and in R, iduronic units, is about 5%; R₁ and R being not simultaneously SO₃⁻ and being both hydrogen in from about 30% to about 40% of the uronic acid units; the sulphation degree being from about 2.5 to about 2.7, the corresponding cation being a chemically or pharmaceutically acceptable one.

A preferred low molecular weight glycosaminoglycan active ingredient of this class is constituted by a mixture of chains with a mean molecular weight of about 7,000, in which at least 90% of said chains have the formula I above, wherein about 55% of the uronic acid units are those of iduronic acid and
- R₃ is about 85% SO₃⁻;
- R₂ is about 20% SO₃⁻;
- R₁ is about 25% SO₃⁻ in iduronic units and 0 to about 5% SO₃⁻ in glucuronic units;
- R is about 30% SO₃⁻ in glucuronic units and 0 to about 5% in iduronic units;
- the sum of the SO₃⁻ percent in R₁, glucuronic units, and in R, iduronic units, is about 5%;

R₁ and R being not simultaneously SO₃⁻ and being both hydrogen in about 40% of the uronic acid units; the sulphation degree being about 2.55, the corresponding cation being a pharmaceutically acceptable one. A particular preferred glycosaminoglycan active ingredient has these characteristics, with a mean molecular weight of 7,400.

Finally the present invention refers to the effective amount of said glycosaminoglycans for the control of the coagulation and for an antithrombotic treatment

Thus, it is a further object of the present invention to provide a method for controlling coagulation in a mammal or for the prevention or treatment of thrombosis, which comprises administering to said mammal, in need of said coagulation control or in need of said prevention or treatment, a pharmacologically effective amount of a C5-epimerised N,O-sulphate K5 glycosaminoglycan obtainable according to the process wherein steps (i)-(vi) above, including the optional depolymerisation and pharmaceutically acceptable salt formation, are performed.

More particularly, said method comprises administering to said mammal a pharmacologically active amount of a glycosaminoglycan constituted by a mixture of chains in which at least 90% of said chains have the formula I as illustrated and specified above.

Preferably, the method of the present invention comprises administering to said mammal a pharmacologically active dose of a pharmaceutical composition as illustrated above.

The following examples illustrate the invention without, however, limiting it

The typical characteristics of molecular weight and biological activity of the glycosaminoglycans are herein indicated in comparison with unfractionated heparin (fourth international standard) and low molecular weight heparin (first international standard).

Molecular weight, in particular the mean molecular weight, is calculated according to Harenberg and De Vries, J. Chromatography, 1983, 261, 287-292 either using a single column (Pharmacia 75HR) or using two columns (BioRad Bio-sil SEC250). The molecular weights of the end product can be different from those of the starting polysaccharides due to the reaction conditions of the process of the invention.

Anti-Xa activity is determined according to D.P. Thomas et al., Thrombosis and Haemostasis, 1981, 45, 214, against the fourth international standard of heparin.

The aPTT test for global anticoagulant activity is carried out according to Andersson et al., Thrombosis Research, 1976, 9, 575.

The test for HCII activity is performed by mixing 20 µl of a solution of 0.05 PEU/ml (Plasma Equivalent Unit) of HCII (Stago) dissolved in water with 80 µl of a solution of the sample under examination at different concentrations and 50 µl of thrombin (0.18 U/ml-Boehringer) in 0.02M tris buffer pH 7.4 containing 0.15 M HCl and 0.1% PEG-6,000. The solution is incubated for 60 seconds at 37°C, then 50 µl of 1 mM chromogenic substrate Spectrozyme (american Diagnostic) are added. The reaction is continuously recorded for 180 seconds with determinations every second at 405 nm using an automatic coagulometer ACL 7000 (Instrumentation Laboratory).

Anti IIa test is performed by mixing 30 µl of a solution containing 0.5 U/ml of ATIII (Chromogenix) dissolved in 0.1M tris buffer pH 7.4 with 30 µl of a solution of the sample under examination at different concentrations and 60 µl of thrombin [5.3 nKat (Units of Enzymatic Activity)/ml-Chromogenix] in 0.1 M tris buffer pH 7.4. The solution is incubated for 70 seconds at 37°C, then 60 µl of 0.5 mM chromogenic substrate S-2238 (Chromogenix) in water are added. The reaction is continuously recorded for 90 seconds with determinations every second at 405 nm using an automatic coagulometer ACL 7000 (Instrumentation Laboratory).

Affinity for ATIII is determined according to Höök et al., FEBS Letters, 1976, 66, 90-93.

### Example 1

An amount of 10 g of K5 polysaccharide, obtained by fermentation as described in the Italian application MI99A001465 (WO 01/02597) with a purity of 80% (Fig.2), is dissolved in deionised water to obtain a 1% solution. Triton X-100 is added to reach a concentration of 5% and the solution is kept at 55°C for 2 hours under stilling. The solution is brought to 75°C and kept at this temperature till a homogeneous turbid system is obtained and then the solution is rapidly cooled to room temperature. During the cooling two phases are formed. Said thermic treatment is repeated twice on the upper phase (organic phase). The aqueous phase containing K5 is finally 1/10 concentrated under reduced pressure and precipitated with acetone or ethanol. The organic phase is discarded.

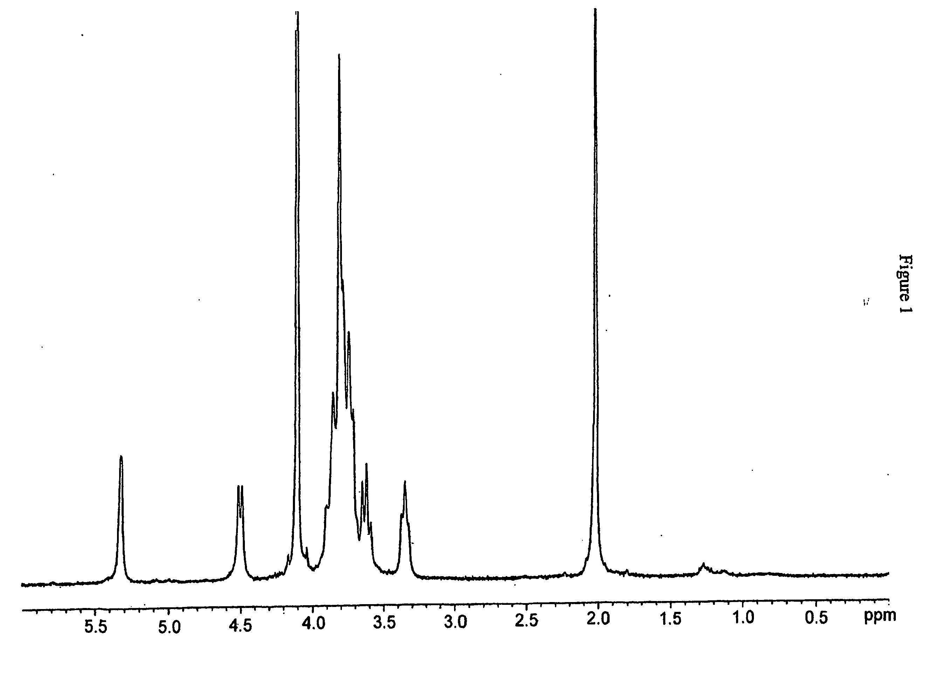

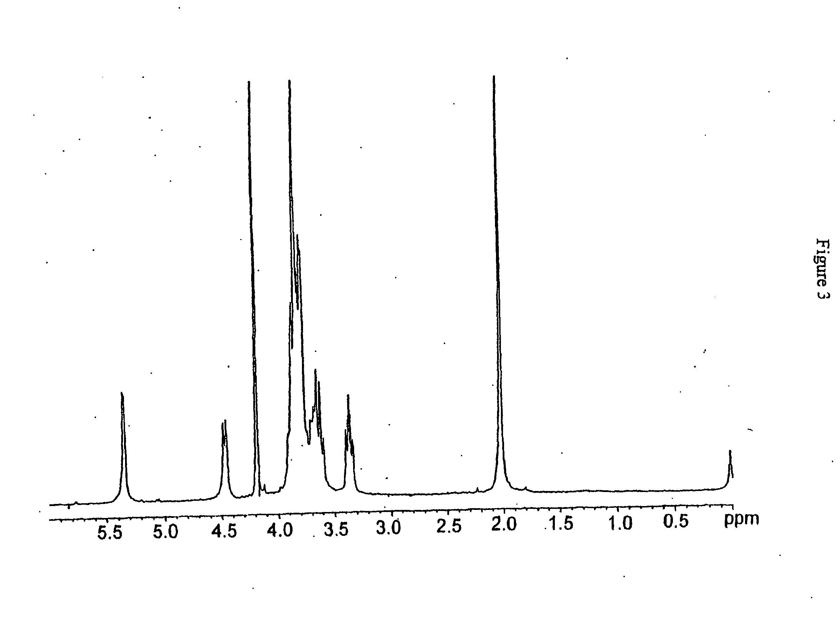

The product obtained is K5 with 90% purity detected by proton NMR (Fig. 3) compared to the spectrum of the working standard (Fig. 1) and a retention time of 9 minutes on the HPLC analysis using two columns (Bio Rad Bio-sil SEC 250).

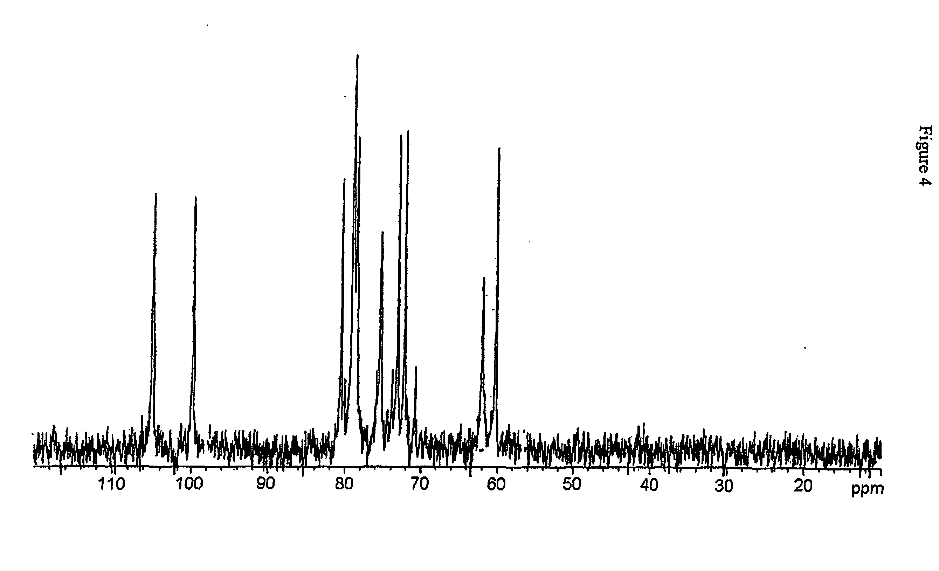

The process proceeds according to the following steps:
(i) The previously purified K5 is dissolved in 1,000 ml of 2 N sodium hydroxide and kept at 60°C for 18 hours. The solution is cooled to room temperature and then brought to neutral pH with 6N hydrochloric acid. N-deacetylated K5 is obtained. The solution containing the N-deacetylated K5 is kept at 40°C and added with 10 gr sodium carbonate in one step and 20 gr. of adduct pyridine.sulfur trioxide in 10 minutes. At the end of the reaction the solution is cooled to room temperature and then brought to pH 7.5-8 with a 5% hydrochloric acid solution.
   The product obtained, N-sulphate K5, is purified from salts by diafiltration using a 1,000 D cut off spiral membrane (prepscale cartridge - Millipore). The purification process is stopped when the conductivity of the permeate is less than 100 µS. The product retained by the membrane is concentrated to 10% polysaccharide using the same diafiltration system and then is freeze dried. The ratio N-sulphate/N-acetyl in the product obtained is 9.5/0.5 measured by carbon 13 NMR (Fig. 4).
(ii) 1 - Preparation of the immobilised C5 epimerase
   To 5 mg of recombinant C5 epimerase obtained according to WO 98/48006, corresponding to 1.2 x 10¹¹ cpm (counts per minutes) dissolved in 200 ml of 25 mM Hepes buffer pH 7.4, containing 0.1 M KCl, 0.1% Triton X-100 and 15 mM ethylenediaminotetracetic acid (EDTA), 100 mg of N-sulphate K5 obtained as described in step (i) are added. The solution is diafiltrated with a 30,000 D membrane at 4°C till disappearance of N-sulphate K5 in the permeate. To the solution retentated by the membrane the buffer is changed by diafiltration against 200 mM NaHCO₃ at pH 7 and, after concentration to 50 ml, 50 ml of CNBr activated Sepharose 4B resin are added and kept to react overnight at 4°C. At the end of the reaction the amount of residual enzyme in the supernatant is measured with the Quantigold method (Diversified Biotec) after centrifugation. The enzyme in the supernatant is absent, showing that with the method described the enzyme is 100% immobilised. To occupy the sites still available the resin is washed with 100 mM tris pH 8. To measure the activity of the immobilised enzyme an amount of immobilised enzyme theoretically correspondent to 1.2 x 10⁷ cpm is loaded into a column. In the column obtained 1 mg of N-sulphate K5 obtained as described in step (i) dissolved in 25 mM Hepes, 0.1M KCl, 0.015 M EDTA, 0.01% Triton X-100, pH 7.4 buffer is dissolved, recirculating it through said column at 37°C overnight at a flow rate of 0.5 ml/minute.

After purification by DEAE chromatographic system and desalting on a Sephadex G-10 the sample is freeze dried and analysed for its content in iduronic acid by proton NMR technique as already described in WO 96/14425.

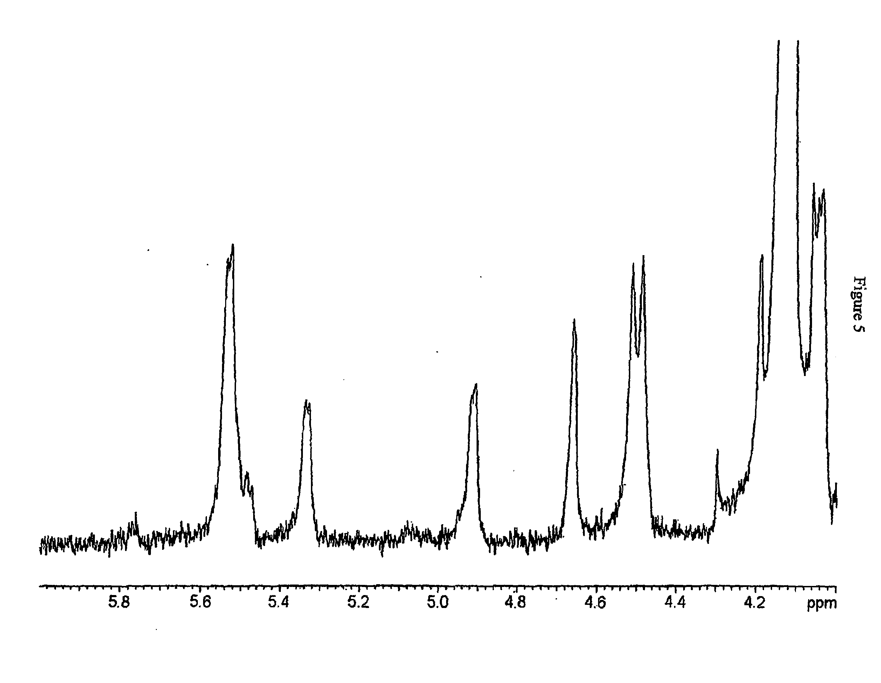

The ratio iduronic acid/glucuronic acid is 30/70 (Fig 5).

### 2 - Epimerisation.

An amount of 10 g of the N-sulphate K5 is dissolved in 600 ml of 25 mM Hepes buffer pH 7 containing 50 mM CaCl₂. The solution obtained is recirculated through a column of 50 ml containing the resin with the immobilised enzyme.

This reaction is performed at 30°C with a flow rate of 200 ml/hour for 24 hours. The product obtained is purified by ultrafiltration and precipitation with ethanol. The pellet is dissolved in water at 10% concentration.

An epimerised product is obtained with a ratio iduronic acid/glucuronic acid 54/46 against a ratio 0/100 of the starting material.

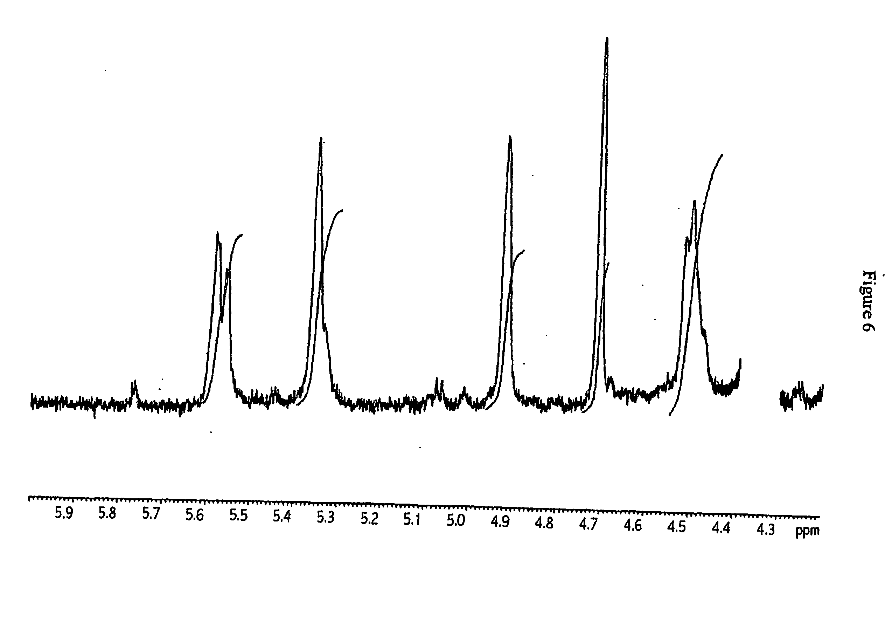

The percentage of epimerisation is calculated by ¹H-NMR (Fig. 6).

The yield calculated measuring the uronic acid content against standard by the carbazole method (Bitter and Muir Anal. Biochem. 39 88-92 (1971)) is 90%.
(iii) The solution containing the epimerised product obtained in step (ii) is cooled to 10°C with a cooling bath and then applied onto a IR 120 H⁺ cationic exchange resin (50 ml). Both the column and the container of the eluted solution are kept at 10°C. After the passage of the solution the resin is washed with 3 volumes of deionised water. The pH of the flow through is more than 6. The acidic solution is brought to neutrality with a 15% aqueous solution of tetrabutylammoniun hydroxide.

The solution is concentrated to 1/10 of the volume in a rotating evaporator under vacuum and freeze-dried. The product is suspended in 200 ml of dimethylformamide (DMF) and added with 150 g of the adduct pyridine.SO₃ dissolved in 200 ml of DMF. The solution is kept at 45°C for 18 hours. At the end of the reaction the solution is cooled to room temperature and added with 1,200 ml of acetone saturated with sodium chloride. The pellet obtained is separated from the solvent by filtration, dissolved with 100 ml of deionised water and sodium chloride is added to 0.2 M concentration. The solution is brought to pH 7.5-8 with 2 N sodium hydroxide and 300 ml of acetone are added. The pellet is separated by filtration. The solid obtained is solubilised with 100 ml deionised water and purified from the residual salts by diafiltration as described in step (i).

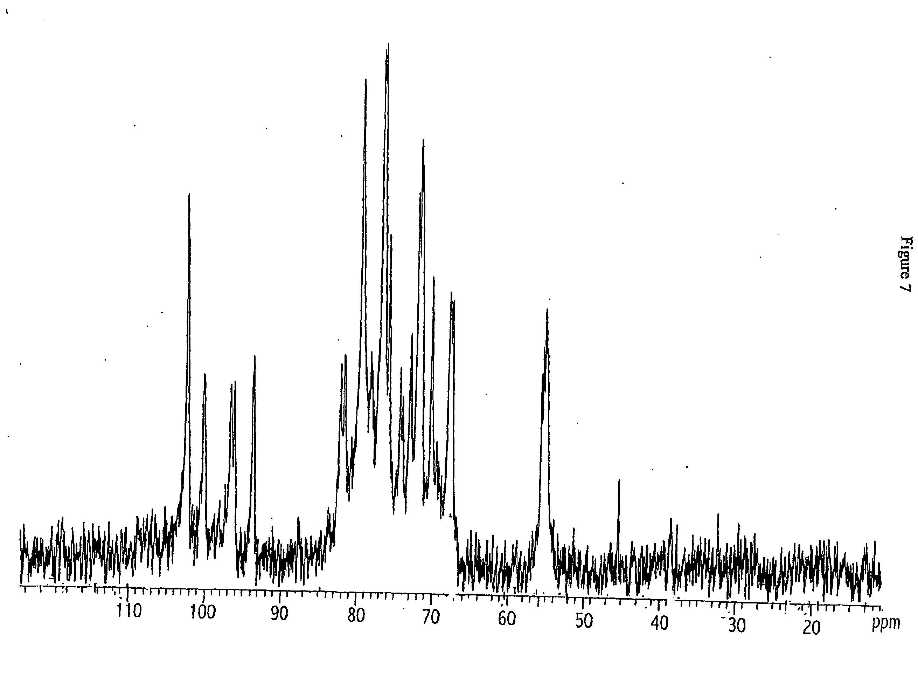

The ¹³C-NMR analysis on a dried small amount of the oversulphated product is shown in Fig. 7.
(iv) The solution containing the product of step (iii) is passed onto a IR 120 H⁺ cationic exchange resin (50 ml). After the passage of the solution the resin is washed with 3 volumes of deionised water. The pH of the flow through is more than 6. The acidic solution is brought to neutrality with pyridine. The solution is concentrated to 1/10 of the volume in a rotating evaporator at 40°C under vacuum and freeze-dried. The product obtained as pyridine salt is added with 500 ml of a solution of DMSO/methanol (9/1 V/V). The solution is kept at 60°C for 2.5 hours and then added with 50 ml deionised water and finally treated with 1,650 ml acetone saturated with sodium chloride. The solid obtained is purified by diafiltration as described in step (i) and a solution at 10% concentration is obtained.

The ¹³C-NMR analysis on a dried small amount in Fig. 8 shows a content of sulphate groups in position 6 of the amino sugar of 20%.
(v) The solution containing the product of step (iv) is passed onto a IR 120 H⁺ cationic exchange resin (50 ml). After the passage of the solution the resin is washed with 3 volumes of deionised water. The pH of the flow through is more than 6. The acidic solution is brought to neutrality with an aqueous solution of 15% tetrabutylammoniun hydroxide. The solution is concentrated to 1/10 of the volume in a rotating evaporator under vacuum and freeze-dried. The product as tetrabutylammonium salt is suspended in 200 ml DMF. The suspension is cooled to 0°C and treated with 40 gr. of the adduct pyridine.SO₃ dissolved in 100 ml DMF. The sulphating agent is added one step. The solution is kept at 0°C for 1.5 hours and then is treated with 750 ml acetone saturated with sodium chloride.

The solid obtained is purified by diafiltration as described in step (i).
(vi) The solution of step (v) is treated as described in step (i) for N-sulphation.

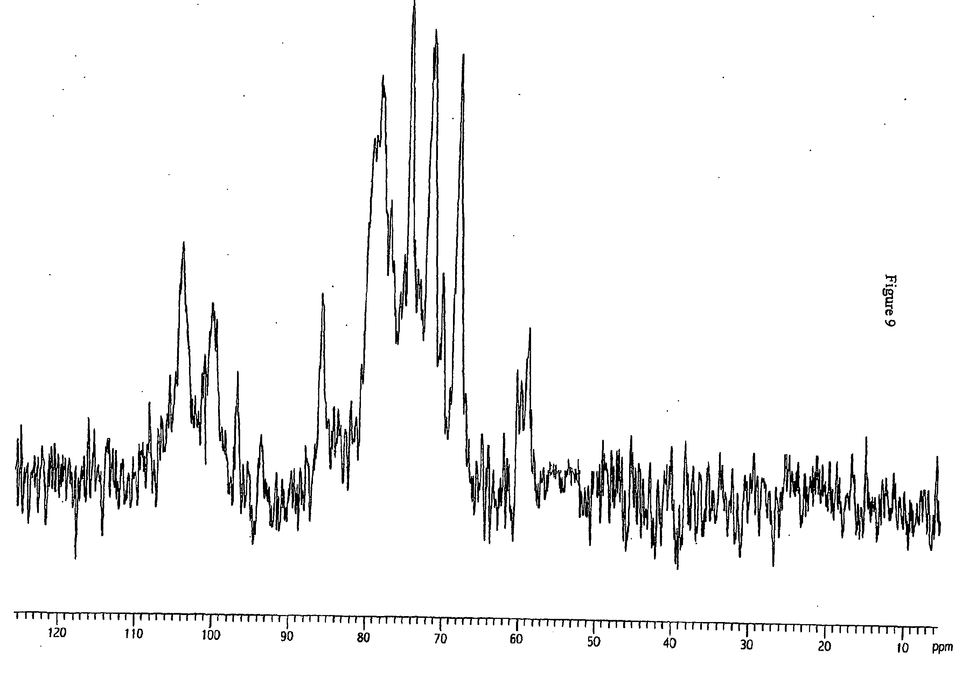

The ¹³C-NMR on a dried small amount of the product obtained is shown in Fig. 9.

The compound obtained shows a mean molecular weight of 15,700 (determined according to Harenberg and De Vries, J. Chromatography, using two columns, BioRad Bio-sil SEC250), sulphate/carboxyl ratio of 2.55, iduronic acid content of 54%, N-sulphate content of >90 %, 6-O sulphate content of 85%, 3-O sulphate glucosamine content of 20%, iduronic acid 2-O-sulphate content of 25%, glucuronic acid 3-O-sulphate content of 30%, no O-disulphated uronic units, unsulphated uronic units content of about 40%. Taking into account the sulphate/carboxyl ratio of 2.55, by difference it is calculated that about 5% of sulphate groups are present in 2-O-sulphate glucuronic acid and 3-O-sulphate iduronic acid units. Furthermore, the compound obtained contains 55 % of an ATIII high affinity fraction and the following in vitro anticoagulant activities compared with those of standard heparin taken as 100: anti-Xa 157, aPTT 78, anti-IIa 373, HCII 161.

### Example 2

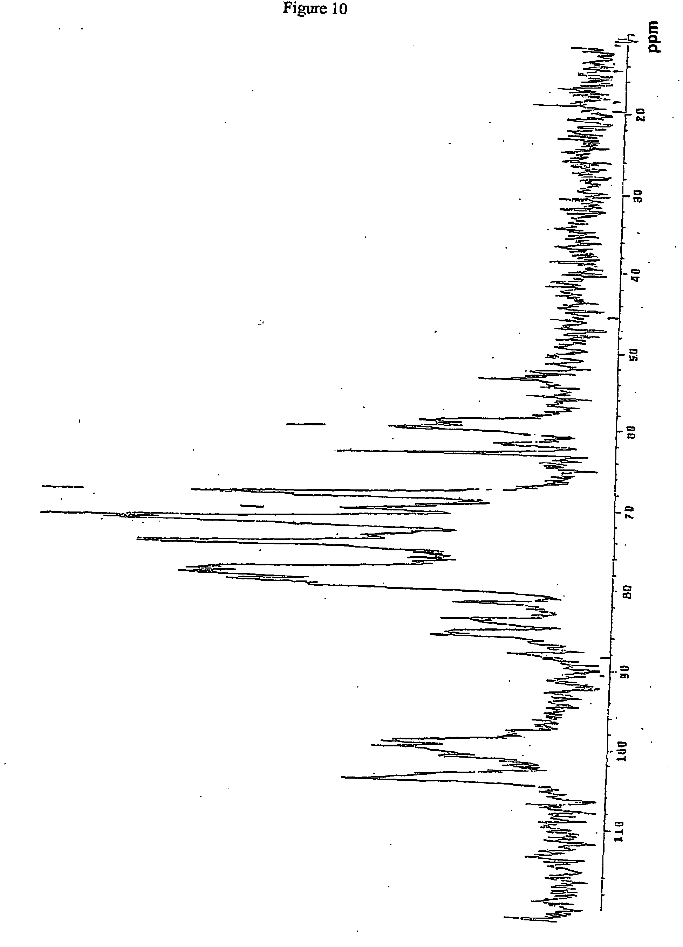

The C5-epimerised N,O-sulphate K5 obtained at the end of step (vi) of Example 1 is depolymerised with nitrous acid under controlled conditions as described in WO 82/03627. More particularly, 5 g of sample are dissolved in 250 ml of water and cooled to 4°C with a thermostatic bath. The pH is brought to 2 with 1N hydrochloric acid previously cooled to 4°C, then 10 ml of a solution of 1% sodium nitrite are added thereinto and, if necessary, the pH is brought to 2 with 1N hydrochloric acid. The mixture is kept under slow stirring for 15 minutes, the solution is neutralised with 1N NaOH, previously cooled to 4°C, then 250 mg of sodium borohydride dissolved in 13 ml of deionised water are added thereinto and the slow stirring is continued for 4 hours. The pH of the mixture is brought to 5 with 1N hydrochloric acid, then said mixture is let to stand under stirring for 10 minutes to destroy the excess of sodium borohydride, and finally neutralised with 1N NaOH. The product is recovered by precipitation with 3 volumes of ethanol and dried in a vacuum oven. In Fig. 10, the ¹³C-NMR spectrum of the compound thus obtained is shown. The compound has a mean molecular weight of 7,400, sulphate/carboxyl ratio of 255, iduronic acid content of 54%, N-sulphate content > 90%, 6-O-sulphate content of 85 %, 3-O-sulphate glucosamine content of 20%, iduronic acid 2-O-sulpbate content of 25%, glucuronic acid 3-O-sulphate content of 30%, no O-disulphated uronic units, unsulphated uronic units content of 40%. Taking into account the sulphate/carboxyl ratio of 2.55, by difference it is calculated that 5% of sulphate groups are present in 2-O-sulphate glucuronic acid and 3-O-sulphate iduronic acid units. Furthermore, the glycosaminoglycan thus obtained contains 34 % of ATIII high affinity fraction and the following in vitro anticoagulant activities compared to those of heparin taken as 100: anti-Xa 99, aPTT 52, anti-IIa 203, HCII 108. In comparison with said activities of the first International Standard of low molecular weight heparin (LMWH), taken as 100, the depolymerised, C5-epimerised N,O-sulphate K5 glycosaminoglycan thus obtained shows the following anticoagulant activities: anti Xa 117, aPTT 173, anti IIa 615 (HCII was not determined for LMWH). These results show that, for the C5-epimerised N,O-sulphate K5 thus obtained,
- anti-IIa/aPTT and anti-IIa/anti-Xa ratios are about four times and, respectively, twice as high as those of standard heparin;
- anti-IIa/aPTT and anti-IIa/anti-Xa ratios are about 3.5 times and, respectively, about five times as high as those of standard LMWH;
   HCII/aPTT and HCII/anti-Xa ratios are about twice and, respectively, about as high as those of standard heparin;
   anti-Xa and HCII activities being about as high as those of standard heparin and aPTT activity being about one half that of standard heparin.

### Example 3

Example 1 is repeated using, in step (ii), the recombinant enzyme obtained as described by Jin-Ping L. et al. (Characterization of D-glucuronosyl-C5 epimerase involved in the biosynthesis of heparin and heparan sulphate. Journal Biological Chemistry, 2001,Vol. 276, 20069-20077). The compound obtained shows a mean molecular weight of 14,900 (determined according to Harenberg and De Vries, J. Chromatography, using two columns, BioRad Bio-sil SEC250)), sulphate/carboxyl ratio of 2.7, iduronic acid content of 54%, N-sulphate content of >90%, 6-O-sulphate content of 90%, 3-O-sulphate glucosamine of 20%, iduronic acid 2-O-sulphate content of 30%, glucuronic acid 3-O-sulphate content of 35%, no O-disulphated uronic units, unsulphated uronic units content of about 30%. Taking into account the sulphate/carboxyl ratio of 2.7, by difference it is calculated that about 5% of sulphate groups are present in 2-O-sulphate glucuronic acid and 3-O-sulphate iduronic units. Furthermore, the compound obtained shows the following in vitro anticoagulant activities compared to those of standard heparin taken as 100: anti-Xa 166, aPTT 76, anti-IIa 400, HCII 283.

Without further elaboration, it is believed that one skilled in the art can, using the preceding description, utilise the present invention to its fullest extent. The preceding preferred specific embodiments are, therefore, to be constructed as merely illustrative, and not limitative of the remainder of the disclosure in any way whatsoever.

## Claims

1. A process for the preparation of K5 glycosaminoglycans comprising the steps of (i) N-deacetylation/N-sulphation of the polysaccharide K5, (ii) partial C5-epimerisation of the carboxyl group of the glucuronic acid moiety to the corresponding iduronic acid moiety, (iii) oversulphation, (iv) selective O-desulphation, (v) optional 6-O-sulphation, and (vi) N-sulphation, in which step (iv) comprises treating the oversulphated product obtained at the end of step (iii) with a mixture methanol/dimethyl sulfoxide for a period of time of from 135 to 165 minutes.

2. A process according to claim 1 in which said period of time is of about 150 minutes.

3. A process according to claim 1 in which said treatment is made for a period of time of 150 minutes at a temperature of 60°C.

4. A process for the preparation of novel glycosaminoglycans, which comprises
(i) reacting polysaccharide K5 with a N-deacetylating agent, then treating the N-deacetylated product with a N-sulphating agent;
(ii) submitting the K5-N-sulphate thus obtained to a C5-epimerisation by glucuronosyl C5 epimerase to obtain a C5-epimerised N-sulphate K5 in which the iduronic/glucuronic ratio is from 60/40 to 40/60;
(iii) converting the C5-epimerised N-sulphate K5, having a content of 40 to 60% iduronic acid over the total uronic acids, into a tertiary or quaternary salt thereof, then treating the salt thus obtained with an O-sulphating agent in an aprotic polar solvent at a temperature of 40-60°C for 10-20 hours;
(iv) treating a salt with an organic base of the O-oversulphated product thus obtained with a mixture dimethyl sulfoxide/methanol at 50-70 °C for 135-165 minutes;
(v) treating a salt with an organic base of the partially O-desulphated product thus obtained with an O-sulphating agent at a temperature of 0-5°C;
(vi) treating the product thus obtained with a N-sulphating agent;
whatever product obtained at the end of one of steps (ii) to (vi) being optionally submitted to a depolymerisation.

5. A process according to claim 4, wherein a previously purified K5 is used as starting material.

6. A process according to one of claims 4 and 5, wherein, in step (i), hydrazine or a salt thereof or an alkaline metal hydroxide is used as a N-deacetylating agent and pyridine.sulphur trioxide or trimethylamine.sulphur trioxide adduct is used as a N-sulphating agent.

7. A process according to one of claims 4-6 wherein, in step (ii), said C5 epimerisation is performed using the enzyme glucuronosyl C5 epimerase in solution or in immobilised form in presence of divalent cations.

8. A process according to claim 7 wherein said divalent cations comprise at least one of Ba, Ca, Mg and Mn.

9. A process according to one of claims 4-8, wherein, in step (ii), said epimerase comprises recombinant glucuronosyl C5 epimerase, glucuronosyl C5 epimerase from murine mastocytoma and glucuronosyl C5 epimerase extracted from bovine liver.

10. A process according to one of claims 4-9 wherein said C5 epimerisation with the enzyme in its immobilised form is performed and comprises recirculating 20-1,000 ml of a solution of 25 mM Hepes at pH of from 6 to 7.4 containing 0.001-10 g of N-sulphated K5 and one of said cations at a concentration between 10 and 60 mM through a column containing from 1.2 x 10⁷ to 3 x 10¹¹cpm of the immobilised enzyme on an inert support.

11. A process according to one of claims 4-9 wherein said pH is of about 7 and said C5 epimerisation is performed with a recombinant enzyme at a temperature of 30°C by recirculating said solution with a flow rate of about 200 ml/hour for a time of 24 hours.

12. A process according to one of claims 4-11, wherein, in step (iii), the pyridine.sulphur trioxide adduct is used as O-sulphating agent.

13. A process according to one of claims 4-12, wherein, in step (iv), the reaction is carried out in dimethyl sulfoxide/methanol 9/1 (V/V) at 60°C for 150 minutes.

14. A process according to one of claims 4-13, wherein a previously purified K5 is used as starting material and, in step (iv), the reaction is carried out in dimethyl sulfoxide/methanol 9/1 (V/V) at 60°C for 150 minutes.

15. A process according to one of claims 4-14, wherein, in step (v), the 6-O-sulphation is carried out at 0-5°C by using the pyridine.sulphur trioxide adduct as O-sulphating agent.

16. A process according to one of claims 4-15, wherein, in step (vi), pyridine.sulphur trioxide or trimethylamine.sulphur trioxide adduct is used as N-sulphating agent.

17. A process according to one of claims 4-16, wherein the product obtained at the end of step (vi) is submitted to a nitrous acid depolymerisation followed by a reduction by sodium borohydride.

18. A process according to one of claims 4-17, wherein a previously purified K5 is used as starting material and, in step (iv), the reaction is carried out in dimethyl sulfoxide/methanol 9/1 (V/V) at 60°C for 150 minutes, and the C5-epimerised N,O-sulphate K5 glycosaminoglycan obtained at the end of step (vi) is submitted to a nitrous acid depolymerisation followed by a reduction by sodium borohydride.

19. A process according to one of claims 4-18, wherein the glycosaminoglycan thus obtained is isolated in form of its sodium salt.

20. A process according to claim 19, wherein said sodium salt is further converted in another salt.

21. A process according to claim 20, wherein said other salt is another alkaline metal, or an alkaline-earth metal, ammonium, (C₁-C₄)trialkylammonium, aluminium or zinc salt

22. A C5-epimerised N,O-sulphate K5 glycosaminoglycan obtainable by a process which comprises
(i) reacting polysaccharide K5 with a N-deacetylating agent, then treating the N-deacetylated product with a N-sulphating agent;
(ii) submitting the N-sulphate K5 thus obtained to a C5-epimerisation by glucuronosyl C5 epimerase to obtain a C5-epimerised N-sulphate K5 in which the iduronic/glucuronic ratio is from 60/40 to 40/60;
(iii) converting the C5-epimerised N-sulphate K5, having a content of 40 to 60% iduronic acid over the total uronic acids, into a tertiary or quaternary salt thereof, then treating the salt thus obtained with an O-sulphating agent in an aprotic polar solvent at a temperature of 40-60°C for 10-20 hours;
(iv) treating a salt with an organic base of the O-oversulphated product thus obtained with a mixture dimethyl sulfoxide/methanol at 50-70 °C for 135-165 minutes;
(v) treating a salt with an organic base of the partially O-desulphated product thus obtained with an O-sulphating agent at a temperature of 0-5°C;
(vi) reacting the product thus obtained with a N-sulphating agent;
whatever product obtained at the end of one of steps (ii) to (vi) being optionally submitted to a depolymerisation and the sodium salt of the end product being optionally converted into another salt.

23. The C5-epimerised N,O-sulphate K5 glycosaminoglycan of claim 22 wherein step (iv)'is carried out in a 9/1 (V/V) dimethyl sulfoxide/methanol mixture at 60°C for 150 minutes.

24. The C5-epimerised N,O-sulphate K5 glycosaminoglycan of claim 22 wherein a previously purified K5 is used as starting material and, in step (iv), the reaction is carried out in dimethyl sulfoxide/methanol 9/1 (V/V) at 60°C for 150. minutes, and the product obtained at the end of step (vi) is submitted to a nitrous acid depolymerisation followed by a reduction by sodium borohydride.

25. A glycosaminoglycan constituted by a mixture of chains in which at least 90% of said chains has the formula I 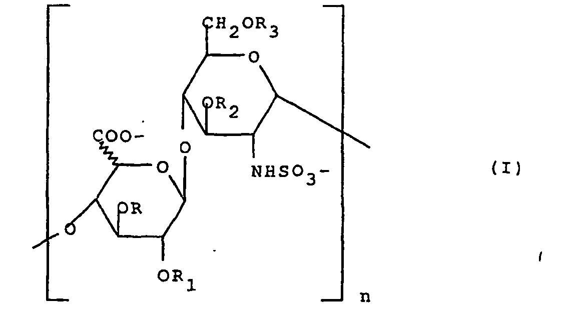 wherein 40-60% of the uronic acid units are those of iduronic acid, n is an integer from 3 to 100, R, R₁, R₂ and R₃ represent a hydrogen atom or a SO₃⁻ group and from about 65% to about 50% of R, R₁, R₂ and R₃ being hydrogen and the remaining being SO₃⁻ groups distributed as follows
- R₃ is from about 85% to about 95% SO₃⁻;
- R₂ is from about 17 to about 21% SO₃⁻;
- R₁ is from about 15 to about 35% SO₃⁻ in iduronic units and 0 to 5% SO₃⁻ in glucuronic units;
- R is from about 20 to about 40% SO₃⁻ in glucuronic units and 0 to 5% SO₃⁻ in iduronic units;
- the sum of the SO₃⁻ percent in R₁, glucuronic units, and in R, iduronic units, is from 3 to 7%;
R₁ and R being not simultaneously SO₃⁻ and being both hydrogen in 25-45% of the uronic acid units; the sulphation degree being from about 2.3 to about 2.9, and the corresponding cation being a chemically or pharmaceutically acceptable one.

26. The glycosaminoglycan of claim 25 wherein said corresponding cation is an alkaline metal, alkaline-earth metal, aluminium or zinc ion.

27. The glycosaminoglycan of claim 25 wherein said corresponding cation is sodium or calcium ion.

28. A glycosaminoglycan according to one of claims 25-27 wherein from about 60% to about 55% of R, R₁, R₂ and R₃ are hydrogen and the remaining are SO₃⁻ groups for a sulphation degree of from about 2.4 to about 2.7.

29. A glycosacunoglycan according to one of claims 25-28 wherein at least 80% of said chains in said mixture of chains have the formula I wherein n is from 3 to 15.

30. A glycosaminoglycan according to claim 29 wherein said chains in said mixture of chains has a molecular weight distribution ranging from 2,000 to 10,000, with a mean molecular weight of from 4,000 to 8,000.

31. A glycosaminoglycan according to claim 30 wherein said chains in said mixture of chains have a mean molecular weight of from 6,000 to 8,000 and at least 90% of said chains has the formula I, wherein 55% of the uronic acid units are those of iduronic acid and R₃ is from 85% to 90% SO₃⁻; R₂ is about 20% SO₃⁻; R₁ is from 25% to 30% SO₃⁻ in iduronic units and 0 to 5% SO₃⁻ in glucuronic units; R is from 30% to 35% SO₃⁻ in glucuronic units and 0 to about 5% SO₃⁻ in iduronic units; the sum of the SO₃⁻ percent in R₁, glucuronic units and in R, iduronic units is 5%; R₁ and R being not simultaneously SO₃⁻ and being both hydrogen in from 30% to 40% of the uronic acid units; the sulphation degree being from 2.5 to 2.7.

32. A glycosaminoglycan according to claim 31 wherein said chains in said mixture of chains have a mean molecular weight of 7,000 and at least 90% of said chains has the formula I, wherein about 55% of the uronic acid units are those of iduronic acid and
- R₃ is 85% SO₃⁻;
- R₂ is 20% SO₃⁻;
- R₁ is 25% SO₃⁻ in iduronic units and 0 to 5% SO₃⁻ in glucuronic units;
- R is 30% SO₃⁻ in glucuronic units and 0 to 5% SO₃⁻ in iduronic units;
- the sum of the SO₃⁻ percent in R₁, glucuronic units and in R, iduronic units, is 5%;
R₁ and R being not simultaneously SO₃⁻ and being both hydrogen in 40% of the uronic acid units; the sulphation degree being 2.55.

33. A glycosaminoglycan according to claim 32, wherein said mixture of chains has a mean molecular weight of 7,400.

34. A glycosaminoglycan according to one of claims 25-28 wherein at least 80% of said chains in said mixture of chains have the formula I wherein n is from 20 to 100.

35. A glycosaminoglycan according to claim 34, wherein said mixture of chains has a molecular weight distribution ranging from 9,000 to 60,000, with a mean molecular weight of from 12,000 to 30,000.

36. A glycosaminoglycan according to claim 35 wherein said chains in said mixture of chains have a mean molecular weight of 14,000-16,000 and at least 90% of said chains has the formula I, wherein 55% of the uronic acid units are those of iduronic acid and
- R₃ is from 85% to 90% SO3⁻;
- R₂ is 20% SO3⁻,
- R₁ is from 25% to 30% SO3⁻ in iduronic units and 0 to 5% SO3⁻ in glucuronic units ;
- R is from 30% to 35% SO3⁻ in glucuronic units and 0 to 5% SO3⁻ in iduronic units;
- the sum of the SO3⁻ percent in RI, glucuronic units and in R, iduronic units, is 5%;
R₁ and R being not simultaneously SO3⁻ and being both hydrogen in from 30% to 40% of the uronic acid units ; the sulphation degree being from 2.5 to 2.7.

37. A glycosaminoglycan according to claim 36, wherein said mixture of chains has a mean molecular weight of 15,700.

38. A pharmaceutical composition comprising a pharmacologically effective amount of a glycosaminoglycan according to one of claims 22-37, as a pharmaceutically acceptable salt thereof, as an active ingredient, and a pharmaceutically acceptable carrier.

39. Use of a glycosaminoglycan according to one of claims 22-37 for the preparation of pharmaceutical compositions for controlling coagulation in a mammal.

40. Use of a glycosaminoglycan according to one of claims 22-37 for the preparation of pharmaceutical compositions for preventing or treating thrombosis in a mammal.

41. Use according to one of claims 39-40 wherein said pharmaceutical composition contains 5 to 100 mg of said glycosaminoglycan.

## Patentansprüche

1. Verfahren zur Herstellung von K5-Glykosaminoglykanen, umfassend die Schritte: (i) N-Deacetylierung/N-Sulfatierung des Polysaccharids K5, (ii) partielle C5-Epimerisierung der Carboxylgruppe der Glucuronsäureeinheit zu der entsprechenden Iduronsäureinheit, (iii) Übersulfatierung, (iv) selektive O-Desulfatierung, (v) optional 6-O-Sulfatierung und (vi) N-Sulfatierung, wobei Schritt (iv) die Behandlung des übersulfatierten Produkts, das am Ende von Schritt (iii) erhalten wird, mit einer Mischung von Methanol/Dimethylsulfoxid für einen Zeitraum von 135 bis 165 Minuten umfasst.

2. Verfahren gemäß Anspruch 1, in dem dieser Zeitraum etwa 150 Minuten beträgt.

3. Verfahren gemäß Anspruch 1, in dem diese Behandlung für einen Zeitraum von 150 Minuten bei einer Temperatur von 60°C erfolgt.

4. Verfahren zur Herstellung von neuen Glykosaminoglykanen, das umfasst:
(i) Umsetzen von Polysaccharid K5 mit einem N-Deacetylierungsmittel, dann Behandeln des N-deacetylierten Produktes mit einem N-Sulfatierungsmittel;
(ii) Unterwerfen des so erhaltenen K5-N-Sulfats einer C5-Epimerisierung durch Glucuronosyl-C5-Epimerase, um C5-epimerisiertes N-Sulfat K5 zu erhalten, in dem das Iduron-/Glucuronsäure-Verhältnis von 60/40 bis 40/60 reicht;
(iii) Umsetzen des C5-epimerisierten N-Sulfats K5 mit einem Gehalt von 40 bis 60% Iduronsäure, bezogen auf die Gesamturonsäuren, zu einem tertiären oder quartären Salz desselben, dann Behandeln des so erhaltenen Salzes mit einem O-Sulfatierungsmittel in einem aprotischen polaren Lösungsmittel bei einer Temperatur von 40-60°C für 10-20 Stunden;
(iv) Behandeln eines Salzes mit einer organischen Base des so erhaltenen O-übersulfatierten Produkts mit einer Mischung von Dimethylsulfoxid/Methanol bei 50-70°C für 135-165 Minuten;
(v) Behandeln eines Salzes mit einer organischen Base des so erhaltenen partiell O-desulfatierten Produkts mit einem O-Sulfatierungsmittel bei einer Temperatur von 0-5°C;
(vi) Behandeln des so erhaltenen Produkts mit einem N-Sulfatierungsmittel;
wobei welches Produkt auch immer, das am Ende von einem der Schritte (ii) bis (vi) erhalten wird, optional einer Depolymerisation unterworfen wird.

5. Verfahren gemäß Anspruch 4, wobei ein zuvor gereinigtes K5 als Ausgangsmaterial verwendet wird.

6. Verfahren gemäß einem der Ansprüche 4 und 5, wobei in Schritt (i), Hydrazin oder ein Salz desselben oder ein Alkalimetallhydroxid als ein N-Deacetylierungsmittel verwendet wird und Pyridin-Schwefeltrioxid- oder Trimethylamin·Schwefeltrioxid-Addukt als ein N-Sulfatierungsmittel verwendet wird.

7. Verfahren gemäß einem der Ansprüche 4-6, wobei in Schritt (ii) diese C5-Epimerisierung unter Verwendung des Enzyms Glucuronosyl-C5-Epimerase in Lösung oder in immobilisierter Form in Gegenwart von zweiwertigen Kationen durchgeführt wird.

8. Verfahren gemäß Anspruch 7, wobei diese zweiwertigen Kationen mindestens eines von Ba, Ca, Mg und Mn umfassen.

9. Verfahren gemäß einem der Ansprüche 4-8, wobei in Schritt (ii) diese Epimerase rekombinante Glucuronosyl-C5-Epimerase, Glucuronosyl-C5-Epimerase aus Maus-Mastocytom und Glucuronosyl-C5-Epimerase aus Rinderleber extrahiert umfasst.

10. Verfahren gemäß einem der Ansprüche 4-9, wobei diese C5-Epimerisierung mit dem Enzym in seiner immobilisierten Form durchgeführt wird und Zirkulieren von 20-1.000 ml einer Lösung von 25 mM Hepes bei einem pH von 6 bis 7,4, die 0,001-10 g N-sulfatiertes K5 und eines dieser Kationen bei einer Konzentration zwischen 10 und 60 mM enthält, durch eine Säule, die 1,2 x 10⁷ bis 3 x 10¹¹ cpm des immobilisierten Enzyms auf einem inerten Träger enthält.

11. Verfahren gemäß einem der Ansprüche 4-9, wobei dieser pH etwa 7 beträgt und diese C5-Epimerisierung mit einem rekombinaten Enzym bei einer Temperatur von 30°C durch Zirkulieren dieser Lösung mit einer Strömungsgeschwindigkeit von etwa 200 ml/h für eine Zeit von 24 Stunden durchgeführt wird.

12. Verfahren gemäß einem der Ansprüche 4-11, wobei in Schritt (iii) das Pyridin·Schwefeltrioxid-Addukt als ein O-Sulfatierungsmittel verwendet wird.

13. Verfahren gemäß einem der Ansprüche 4-12, wobei in Schritt (iv) die Reaktion in Dimethylsulfoxid/Methanol 9/1 (V/V) bei 60°C für 150 Minuten durchgeführt wird.

14. Verfahren gemäß einem der Ansprüche 4-13, wobei ein zuvor gereinigtes K5 als Ausgangsmaterial verwendet wird und in Schritt (iv) die Reaktion in Dimethylsulfoxid/Methanol 9/1 (V/V) bei 60°C für 150 Minuten durchgeführt wird.

15. Verfahren gemäß einem der Ansprüche 4-14, wobei in Schritt (v) die 6-O-Sulfatierung bei 0-5°C unter Verwendung des Pyridin·Schwefeltrioxid-Addukts als O-Sulfatierungsmittel durchgeführt wird.

16. Verfahren gemäß einem der Ansprüche 4-15, wobei in Schritt (vi) Pyridin·Schwefeltrioxid oder Trimethylamin·Schwefeltrioxid-Addukt als ein N-Sulfatierungsmittel verwendet wird.

17. Verfahren gemäß einem der Ansprüche 4-16, wobei das Produkt, das am Ende von Schritt (vi) erhalten wird, einer Salpetrigsäure-Depolymerisation, gefolgt von einer Reduktion mit Natriumborhydrid, unterworfen wird.

18. Verfahren gemäß einem der Ansprüche 4-17, wobei ein zuvor gereinigtes K5 als Ausgangsmaterial verwendet wird und in Schritt (iv) die Reaktion in Dimethylsulfoxid/Methanol 9/1 (V/V) bei etwa 60°C für 150 Minuten durchgeführt wird und das C5-epimerisierte N,O-sulfatierte K5-Glykosaminoglykan, das am Ende von Schritt (vi) erhalten wird, einer Salpetrigsäure-Depolymerisation, gefolgt von einer Reduktion mit Natriumborhydrid, unterworfen wird.

19. Verfahren gemäß einem der Ansprüche 4-18, wobei das so erhaltene Glykosaminoglykan in Form seines Natriumsalzes isoliert wird.

20. Verfahren gemäß Anspruch 19, wobei dieses Natriumsalz weiter in ein anderes Salz umgewandelt wird.

21. Verfahren gemäß Anspruch 20, wobei dieses andere Salz ein anderes Alkalimetall- oder Erdalkalimetall-, Ammonium-, (C₁-C₄)-Trialkylammonium-, Aluminium- oder Zinksalz ist.

22. C5-epimerisiertes N,O-sulfatiertes K5-Glykosaminoglykan, erhältlich durch ein Verfahren, das umfasst:
(i) Umsetzen von Polysaccharid K5 mit einem N-Deacetylierungsmittel, dann Behandeln des N-deacetylierten Produkts mit einem N-Sulfatierungsmittel;
(ii) Unterwerfen des so erhaltenen N-Sulfats K5 einer C5-Epimerisierung durch Glucuronosyl-C5-Epimerase, um ein C5-epimerisiertes N-Sulfat K5 zu erhalten, in dem das Iduron-/Glucuronsäure-Verhältnis von 60/40 bis 40/60 reicht;
(iii) Umsetzen des C5-epimerisierten N-Sulfats K5 mit einem Gehalt von 40 bis 60% Iduronsäure, bezogen auf die Gesamturonsäuren, zu einem tertiären oder quartären Salz desselben und dann Behandeln des so erhaltenen Salzes mit einem O-Sulfatierungsmittel in einem aprotischen polaren Lösungsmittel bei einer Temperatur von 40-60°C für 10-20 Stunden;
(iv) Behandeln eines Salzes mit einer organischen Base des so erhaltenen O-übersulfatierten Produkts mit einer Mischung von Dimethylsulfoxid/Methanol bei 50-70°C für 135-165 Minuten;
(v) Behandeln eines Salzes mit einer organischen Base des so erhaltenen partiell O-desulfatierten Produkts mit einem O-Sulfatierungsmittel bei einer Temperatur von 0-5°C;
(vi) Umsetzen des so erhaltenen Produkts mit einem N-Sulfatierungsmittel;
wobei welches Produkt auch immer, das am Ende von einem der Schritte (ii) bis (vi) erhalten wird, optional einer Depolymerisation unterworfen wird und das Natriumsalz des Endprodukts optional in ein anderes Salz umgewandelt wird.

23. C5-epimerisiertes N,O-Sulfat-K5-Glykosaminoglykan nach Anspruch 22, wobei Schritt (iv) in einer 9/1 (V/V) Dimethylsulfoxid/Methanol-Mischung bei 60°C für 150 Minuten durchgeführt wird.

24. C5-epimerisiertes N,O-Sulfat-K5-Glykosaminoglykan nach Anspruch 22, wobei ein zuvor gereinigtes K5 als Ausgangsmaterial verwendet wird und in Schritt (iv) die Reaktion in Dimethylsulfoxid/Methanol 9/1 (V/V) bei 60°C für 150 Minuten durchgeführt wird und das Produkt, das am Ende von Schritt (vi) erhalten wird, einer Salpetrigsäure-Depolymerisation, gefolgt von einer Reduktion mit Natriumborhydrid, unterworfen wird.

25. Glykosaminoglykan, das aus einer Mischung von Ketten gebildet ist, in welcher mindestens 90% dieser Ketten die Formel I aufweisen 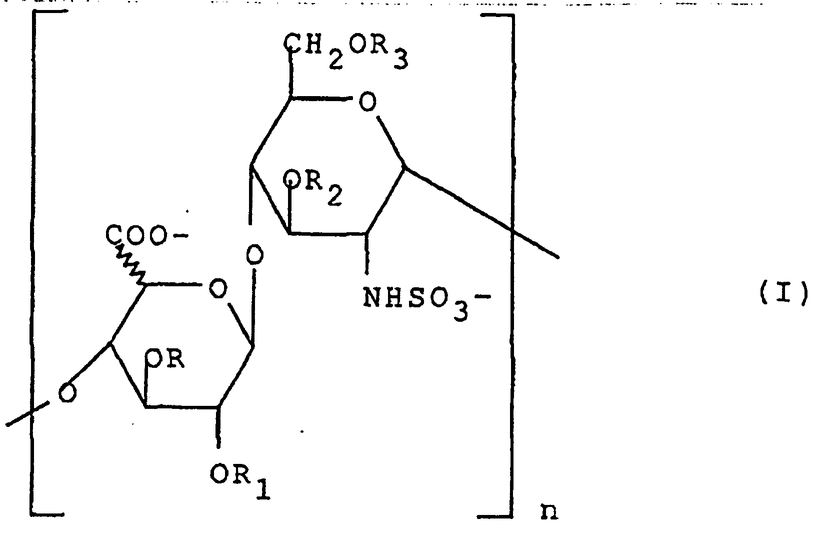 worin 40-60% der Uronsäureeinheiten die von Iduronsäure sind, n eine ganze Zahl von 3 bis 100 ist, R, R₁, R₂ und R₃ ein Wasserstoffatom oder eine SO₃⁻-Gruppe darstellen und etwa 65 bis etwa 50% von R, R₁, R₂ und R₃ Wasserstoff sind und die Verbleibenden SO₃⁻-Gruppen sind, die wie folgt verteilt sind:
- R₃ ist zu etwa 85 bis etwa 95% SO₃⁻;
- R₂ ist zu etwa 17 bis etwa 21 % SO₃⁻;
- R₁ ist zu etwa 15 bis etwa 35% SO₃⁻ in Iduronsäureeinheiten und zu 0 bis 5% SO₃⁻ in Glucuronsäureeinheiten;
- R ist zu etwa 20 bis etwa 40% SO₃⁻ in Glucuronsäureeinheiten und zu 0 bis 5% SO₃⁻ in Iduroneinheiten;
- die Summe der SO₃⁻, die in R¹, Glucuronsäureeinheiten, und in R, Iduronsäureeinheiten, vorhanden sind, beträgt 3 bis 7%;
wobei R₁ und R nicht gleichzeitig SO₃⁻ sind und in 25-45% der Uronsäureeinheiten beide Wasserstoff sind; der Sulfatierungsgrad von etwa 2,3 bis etwa 2,9 reicht und das entsprechende Kation ein chemisch oder pharmazeutisch akzeptables ist.

26. Glykosaminoglykan nach Anspruch 25, wobei dieses korrespondierende Kation ein Alkalimetall-, Erdalkalimetall-, Aluminium- oder Zinkion ist.

27. Glykosaminoglykan nach Anspruch 25, wobei dieses korrespondierende Kation Natrium- oder Calciumion ist.

28. Glykosaminoglykan gemäß einem der Ansprüche 25-27, wobei etwa 60 bis etwa 55% von R, R₁, R₂ und R₃ Wasserstoff sind und die Verbleibenden SO₃⁻ Gruppen für einen Sulfatierungsgrad von etwa 2,4 bis etwa 2,7 sind.

29. Glykosaminoglykan gemäß einem der Ansprüche 25-28, wobei mindestens 80% dieser Ketten in dieser Mischung von Ketten die Formel I aufweisen, worin n gleich 3 bis 15 ist.

30. Glykosaminoglykan gemäß Anspruch 29, wobei diese Ketten in dieser Mischung von Ketten eine Molekulargewichtsverteilung im Bereich von 2.000 bis 10.000 aufweisen, mit einem mittleren Molekulargewicht von 4.000 bis 8.000.

31. Glykosaminoglykan gemäß Anspruch 30, wobei diese Ketten in dieser Mischung von Ketten ein mittleres Molekulargewicht von 6.000 bis 8.000 aufweisen und mindestens 90% dieser Ketten die Formel I aufweisen, wobei 55% der Uronsäureeinheiten die von Iduronsäure sind und R₃ zu 85 bis 90% SO₃⁻ ist; R₂ zu etwa 20% SO₃⁻ ist; R₁ zu 25 bis 30% SO₃⁻ in Iduronsäureeinheiten und zu 0 bis 5% SO₃⁻ in Glucuronsäureeinheiten ist; R zu 30 bis 35% SO₃⁻ in Glucuronsäureeinheiten und zu 0 bis etwa 5% SO₃⁻ in Iduronsäureeinheiten ist; die Summe der SO₃⁻, die in R₁. Glucuronsäureeinheiten, und in R, Iduronsäureeinheiten, vorhanden sind, etwa 5% beträgt; R₁ und R nicht gleichzeitig SO₃⁻ sind und beide in etwa 30 bis 40% der Uronsäureeinheiten Wasserstoff sind; der Sulfatierungsgrad von 2,5 bis 2,7 reicht.

32. Glykosaminoglykan gemäß Anspruch 31, wobei diese Ketten in dieser Mischung von Ketten ein mittleres Molekulargewicht von 7.000 aufweisen und mindestens 90% dieser Ketten die Formel I aufweisen, wobei etwa 55 % der Uronsäureeinheiten die von Iduronsäure sind und
- R₃ zu 85% SO₃⁻ ist;
- R₂ zu 20% SO₃⁻ ist;
- R₁ zu 25% SO₃⁻ in Iduronsäureeinheiten und zu 0 bis 5% SO₃⁻ in Glucuronsäureeinheiten ist;
- R zu 30% SO₃⁻ in Glucuronsäureeinheiten und zu 0 bis 5% SO₃⁻ in Iduronsäureeinheiten ist;
- die Summe der SO₃⁻, die in R₁, Glucuronsäureeinheiten, und in R, Iduronsäureeinheiten, vorhanden sind, 5% beträgt;
- R₁ und R nicht gleichzeitig SO₃⁻ sind und beide in 40% der Uronsäureeinheiten Wasserstoff sind; der Sulfatierungsgrad 2,55 beträgt.

33. Glykosaminoglykan gemäß Anspruch 32, wobei diese Mischung von Ketten ein mittleres Molekulargewicht von 7.400 aufweist.

34. Glykosaminoglykan gemäß einem der Ansprüche 25-28, wobei mindestens 80% dieser Ketten in dieser Mischung von Ketten die Formel I aufweisen, worin n von 20 bis 100 reicht.

35. Glykosaminoglykan gemäß Anspruch 34, wobei diese Mischung von Ketten eine Molekulargewichtsverteilung im Bereich von 9.000 bis 60.000 aufweist, mit einem mittleren Molekulargewicht von 12.000 bis 30.000.

36. Glykosaminoglykan gemäß Anspruch 35, wobei diese Ketten in dieser Mischung von Ketten ein mittleres Molekulargewicht von 14.000-16.000 aufweisen und mindestens 90% dieser Ketten die Formel I aufweisen, wobei etwa 55% der Uronsäureeinheiten die von Iduronsäure sind und
- R₃ zu 85 bis 90% SO₃⁻ ist;
- R₂ zu 20% SO₃⁻ ist;
- R₁ zu 25 bis 30% SO₃⁻ in Iduronsäureeinheiten und zu 0 bis 5% SO₃⁻ in Glucuronsäureeinheiten ist;
- R zu 30 bis 35% SO₃⁻ in Glucuronsäureeinheiten und zu 0 bis 5% SO₃⁻ in Iduronsäureeinheiten ist;
- die Summe der SO₃⁻, die in R₁, Glucuronsäureeinheiten, und in R, Iduronsäureeinheiten, vorhanden sind, 5% beträgt,
R₁ und R nicht gleichzeitig SO₃⁻ sind und beide in 30 bis etwa 40% der Uronsäureeinheiten Wasserstoff sind; der Sulfatierungsgrad von 2,5 bis etwa 2,7 reicht.

37. Glykosaminoglykan gemäß Anspruch 36, wobei diese Mischung von Ketten ein mittleres Molekulargewicht von 15.700 aufweist.

38. Pharmazeutische Zusammensetzung, enthaltend eine pharmakologisch wirksame Menge eines Glykosaminoglykans gemäß einem der Ansprüche 22-37 als ein pharmazeutisch akzeptables Salz desselben als einen aktiven Bestandteil und einen pharmazeutisch akzeptablen Träger.

39. Verwendung eines Glykosaminoglykans gemäß einem der Ansprüche 22-37 zur Herstellung von pharmazeutischen Zusammensetzungen zur Regulierung der Koagulation in einem Säugetier.

40. Verwendung eines Glykosaminoglykans gemäß einem der Ansprüche 22-37 zur Herstellung von pharmazeutischen Zusammensetzungen zur Verhinderung oder Behandlung von Thrombosen in einem Säugetier.

41. Verwendung gemäß einem der Ansprüche 39-40, wobei diese pharmazeutische Zusammensetzung 5 bis 100 mg dieses Glykosaminoglykans enthält.

## Revendications

1. Un procédé pour la preparation de K5 glycosaminoglycanes comprenant les passages de (i) N-déacétylation/N-sulfatation du polysaccharide K5, (ii) C5-épimérisation partielle du groupe carboxyle de l'unité acide glucuronique dans l'unité correspondante acide iduronique, (iii) sursulfatation, (iv) O-désulfatation sélective, (v) 6-O-sulfatation éventuelle, et (vi) N-sulfatation, **caractérisé en ce que** le passage (iv) comprend le traitment du produit sursulfaté obtenu à la fin du passage (iii) avec un mélange méthanol/diméthylsulfoxyde pendant une période de temps de 135 à 165 minutes.

2. Procédé selon la revendication 1, **caractérisé en ce que** ladite période de temps est d'environ 150 minutes.

3. Procédé selon la revendication 1, **caractérisé en ce que** ledit traiment est effectué pour une période de temps de 150 minutes à une température de 60°C.

4. Un procédé pour la preparation de nouveaux K5 glycosaminoglycanes, **caractérisé en ce que**
(i) le polysaccharide K5 est fait réagir avec un agent de N-déacétylation, puis le produit N-déacétylé est traité avec un agent de N-sulfatation;
(ii) le K5-N-sulfate ainsi obtenu est soumis at une C5-épimérisation par glucuronosyl C5 épimérase pour obtenir un K5 N-sulfate C5-épimérisé dans lequel le rapport iduronique/glucuronique est de 60/40 à 40/60;
(iii) le K5 N-sulfate C5-épimérisé, ayant un contenu de 40 à 60% en acide iduronique sur le total des acides uroniques, est transformé dans un de ses sels tertiaires ou quaternaires, puis le sel ainsi obtenu est traité avec un agent de O-sulfatation dans un solvant aprotique polaire à une température de 40-60°C pendant 10-20 heures;
(iv) un sel avec une base organique du produit O-sursulfaté ainsi obtenu est traité avec un mélange diméthylsulfoxyde/méthanol à 50-70 °C pendant 135-165 minutes;
(v) un sel avec une base organique du produit partiellement O-désulfaté ainsi obtenu est traité avec un agent de O-sulfatation à une température de 0-5°C;
(vi) le produit ainsi obtenu est traité avec un agent de N-sulfatation;
tout produit obtenu à la fin d'un des passages (ii) à (vi) étant éventuellement soumis à une dépolymérisation.

5. Un procédé selon la revendication 4, **caractérisé en ce que**, comme matière de départ, on utilise un K5 précédemment purifié.

6. Un procédé selon une des revendications 4 et 5, **caractérisé en ce que**, dans le passage (i), on utilise hydrazine ou un de ses sels ou bien un hydroxyde de métal alcalin en tant qu'agent de N-déacétylation et le produit d'addition pyridine.anhydride sulfurique ou triméthylamine.anhydride sulfurique en tant qu'agent de N-sulfatation.

7. Un procédé selon une des revendications 4-6, **caractérisé en ce que**, dans le passage (ii), ladite C5 épimérisation est effectuée en utilisat l'enzyme glucuronosyl C5 épimérase en solution ou sous forme immobilisée en présence de cations bivalents.

8. Un procédé selon la revendication 7, **caractérisé en ce que** lesdits cations bivalents comprennent au moins un des cations Ba, Ca, Mg et Mn.

9. Un procédé selon une des revendications 4-8, **caractérisé en ce que**, dans le passage (ii), ladite épimérase comprend glucuronosyl C5 épimérase recombinante, glucuronosyl C5 épimérase de mastocytome murin et glucuronosyl C5 épimérase extraite de foie bovin.

10. Un procédé selon une des revendications 4-9, **caractérisé en ce que** ladite C5 épimérisation est effectuée avec l'enzyme dans sa forme immobilisée et comprend la recirculation de 20-1,000 ml d'une solution de 25 mM Hepes à un pH de 6 à 7,4 contenant 0,001-10 g de K5 N-déacetylé N-sulfaté et un desdits cations à une concentration entre 10 et 60 mM à travers une colonne contenant de 1,2 x 10⁷ to 3 x 10¹¹ cpm d'enzyme immobilisée sur un support inerte.

11. Un procédé selon une des revendications 4-9, **caractérisé en ce que** ledit pH est d'environ 7 et ladite C5 épimérisation est conduite à une température de 30°C en faisant recirculer ladite solution avec un débit d'environ 200 ml/heure pendant un temps de 24 heures.

12. Un procédé selon une des revendications 4-11, **caractérisé en ce que**, dans le passage (iii), on utilise le produit d'addition pyridine.anhydride sulfurique en tant qu'agent de O-sulfatation.

13. Un procédé selon une des revendications 4-12, **caractérisé en ce que**, dans le passage (iv), la réaction est effectuée dans diméthylsulfoxyde/méthanol 9/1 (V/V) à 60°C pendant 150 minutes.

14. Un procédé selon une des revendications 4-13, **caractérisé en ce qu'**on utilise un K5 précédemment purifié en tant que matière de départ et, dans le passage (iv), on conduit la réaction dans diméthylsulfoxide/méthanol 9/1 (V/V) à 60°C pendant 150 minutes.

15. Un procédé selon une des revendications 4-14, **caractérisé en ce que**, dans le passage (v), la 6-O-sulfatation est effectuée à 0-5°C en utilisant le produit d'addition pyridine.anhydride sulfurique en tant qu'agent de O-sulfatation.

16. Un procédé selon une des revendications 4-15, **caractérisé en ce que**, dans le passage (vi), on utilise le produit d'addition pyridine.anhydride sulfurique ou triméthylamine.anhydride sulfurique en tant qu'agent de N-sulfatation.

17. Un procédé selon une des revendications 4-16, **caractérisé en ce que** le produit obtenu à la fin du passage (vi) est soumis à une dépolymérisation par l'acide nitreux suivie par une réduction avec borohydrure de sodium.

18. Un procédé selon une des revendications 4-17, **caractérisé en ce qu'**on utilise un K5 précédemment purifié en tant que matière de départ et, dans le passage (iv), on conduit la réaction dans diméthylsulfoxyde/methanol 9/1 (V/V) à 60°C pendant 150 minutes, et on soumet le glycosaminoglycane K5-N,O-sulfate C5-épimérisé obtenu à la fin du passage (vi) à une dépolymérisation par l'acide nitreux suivie par une réduction avec borohydrure de sodium.

19. Un procédé selon une des revendications 4-18, **caractérisé en ce que** le glycosaminoglycane ainsi obtenu est isolé sous la forme de son sel de sodium.

20. Un procédé selon la revendication 19, **caractérisé en ce que** ledit sel de sodium est par la suite transformé dans un autre sel.

21. Un procédé selon la revendication 20, **caractérisé en ce que** ledit autre sel est celui d'un autre metal alcalin, ou un sel d'un metal alkalino-terreux, d'ammonium, de (C₁-C₄)trialkylammonium, d'aluminium ou de zinc.

22. Un K5 glycosaminoglycane N,O-sulfate C5-épimérisé, susceptible d'être obtenu par un procédé qui comprend
(i) faire réagir le polysaccharide K5 avec un agent de N-déacétylation, puis traiter le produit N-déacetylé avec un agent de N-sulfatation;
(ii) soumettre le K5-N-sulfate ainsi obtenu à une C5-épimérisation par glucuronosyl C5 épimérase pour obtenir un K5-N-sulfate C5-épimérisé dans lequel le rapport iduronique/glucuronique est de 60/40 à 40/60;
(iii) convertir le K5-N-sulfate C5-épimérisé, ayant un contenu d'acide iduronique de 40 à 60% sur le total des acides uroniques, dans un de ses sels tertiaires or quaternaires, puis traiter le sel ainsi obtenu avec un agent de O-sulfatation dans un solvant aprotique polaire à une température de 40-60°C pendant 10-20 heures;
(iv) traiter un sel de base organique du produit O-sursulfaté ainsi obtenu avec un mélange diméthylsulfoxyde/méthanol à 50-70 °C pendant 135-165 minutes;
(v) traiter un sel de base organique du produit partiellement O-désulfaté ainsi obtenu avec un agent de O-sulfatation à une température de 0-5°C;
(vi) faire réagir le produit ainsi obtenu avec un agent de N-sulfatation;
tout produit obtenu à la fin d'un des passages (ii) to (vi) étant éventuellement soumis à une dépolymérisation et le sel de sodium du produit final étant éventuellement converti dans un autre sel.

23. Le glycosaminoglycane K5 N,O-sulfate C5-épimérisé de la revendication 22, **caractérisé en ce que** le passage (iv) est conduit dans un mélange diméthylsulfoxyde/méthanol 9/1 (V/V) à 60°C pendant 150 minutes.

24. Le glycosaminoglycane K5-N,O-sulfate C5-épimérisé de la revendication 22, **caractérisé en ce que**, comme matière de départ, on utilise un K5 précédemment purifié et, dans le passage (iv), on conduit la réaction dans diméthylsulfoxyde/méthanol 9/1 (V/V) à 60°C pendant 150 minutes, et on soumet le produit obtenu à la fin du passage (vi) à une dépolymérisation par l'acide nitreux suivie par une réduction avec borohydrure de sodium.

25. Un glycosaminoglycane constitué par un mélange de chaînes où au moins 90% desdites chaînes a la formule I 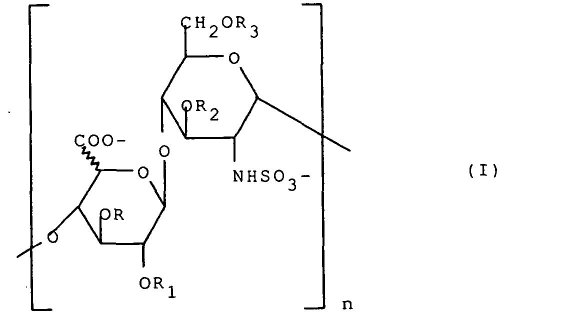 où 40-60% des unités acide uronique son celles de l'acide iduronique, n est un numéro entier de 3 à 100, R, R₁, R₂ et R₃ représentent un atome d'hydrogène ou un groupe SO₃⁻ et d'environ 65% à environ 50% des R, R₁, R₂ et R₃ étant hydrogène et les autres des groupes SO₃⁻ distribués comme il suit
- R₃ est d'environ 85% à environ 95% SO₃⁻;
- R₂ est d'environ 17 à environ 21% SO₃⁻;
- R₁ est d'environ 15 à environ 35% SO₃⁻ dans les unités iduroniques et 0 à 5% SO₃⁻ dans les unités glucuroniques;
- R est d'environ 20 à environ 40% SO₃⁻ dans les unités glucuroniques et 0 à 5% SO₃⁻ dans les unités iduroniques;
- la somme du pour-cent des SO₃⁻ dans R₁, unités glucuroniques, et dans R, unités iduroniques, est de 3 à 7%;
R₁ et R n'étant pas simultanément SO₃⁻ et les deux étant hydrogène dans 25-45% des unités acide uronique; le degré de sulfatation étant d'environ 2,3 à environ 2,9, et le cation correspondant étant chimiquement ou pharmaceutiquement acceptable.

26. Le glycosaminoglycane de la revendication 25, **caractérisé en ce que** ledit cation correspondant est un ion de metal alcalin, de metal alcalino-terreux, d'aluminium ou de zinc.

27. Le glycosaminoglycane de la revendication 25, **caractérisé en ce que** ledit cation correspondant est le ion de sodium ou de calcium.

28. Un glycosaminoglycane selon une des revendications 25-27, **caractérisé en ce que** d'environ 60% à environ 55% des R, R₁, R₂ and R₃ sont hydrogène et ce qui reste sont des groupes SO₃⁻ pour un degré de sulfatation d'environ 2,4 à environ 2,7.

29. Un glycosaminoglycane selon une des revendications 25-28, **caractérisé en ce qu'**au moins 80% desdites chaînes dans ledit mélange de chaînes a la formule 1 où n est de 3 à 15.

30. Un glycosaminoglycane selon la revendication 29, **caractérisé en ce que** lesdites chaînes dans ledit mélange de chaînes a une distribution de poids moléculaires se situant de 2.000 à 10.000, avec un poids moléculaire moyen de 4.000 à 8.000.

31. Un glycosaminoglycane selon la revendication 30, **caractérisé en ce que** lesdites chaînes dans ledit mélange de chaînes ont un poids moléculaire moyen de 6000 à 8000 et au moins 90% desdites chaînes a la formule I, où 55% des unités acide uronique sont celles de l'acide iduronique et R₃ est de 85% à 90% SO₃⁻; R₂ est 20% SO₃⁻; R₁ est de 25% à 30% SO₃⁻ dans les unités iduroniques et de 0 à 5% SO₃⁻ dans les unités glucuroniques; R est de 30% à 35% SO₃⁻ dans les unités glucuroniques et de 0 à 5% SO₃⁻ dans les unités iduroniques; la somme du pour-cent de SO₃⁻ dans R₁, unités glucuroniques et dans R, unités iduroniques, est 5%; R₁ et R n'étant pas simultanément SO₃⁻ et les deux étant hydrogène dans 30% à 40% des unités acide uronique; le degré de sulfatation étant de 2,5 à 2,7.

32. Un glycosaminoglycane selon la revendication 31, **caractérisé en ce que** lesdites chaînes dans ledit mélange de chaînes ont un poids moléculaire moyen de 7000 et at au moins 90% desdites chaînes a la formule I, où 55% des unités acide uronique son celles de l'acide iduronique et
- R₃ est 85% SO₃⁻;
- R₂ est 20% SO₃⁻;
- R₁ est 25% SO₃⁻ dans les unités iduroniques et de 0 à 5% SO₃⁻ dans les unités glucuroniques;
- R est 30% SO₃⁻ dans les unités glucuroniques and 0 à 5% dans les unités iduroniques;
- la somme du pour-cent des SO₃⁻ dans R₁, unités glucuroniques and dans R, unités iduroniques, est 5%;
R₁ et R n'étant pas simultanément SO₃⁻ et les deux étant hydrogène dans 40% des unités acide uronique; le degré de sulfatation étant 2,55.

33. Un glycosaminoglycane selon la revendication 32, **caractérisé en ce que** ledit mélange de chaînes a un poids moléculaire moyen de 7400.

34. Un glycosaminoglycane selon une des revendications 25-28, **caractérisé en ce qu'**au moin 80% desdites chaînes dans ledit mélange de chaînes ont la structure 1 où n est de 20 à 100.

35. Un glycosaminoglycane selon la revendication 34, **caractérisé en ce que** ledit mélange de chaînes a un distribution de poids moléculaires se situant de 9.000 à 60.000, avec un poids moléculaire moyen de 12.000 à 30.000.

36. Un glycosaminoglycane selon la revendication 35 **caractérisé en ce que** lesdites chaînes dans ledit mélange de chaînes a un poids moléculaire moyen de 14.000-16.000 et au moins 90% desdites chaînes a la formule I, où environ 55% des unités acide uronique sont celles de l'acide iduronique et
- R₃ est de 85% à 90% SO₃⁻;
- R₂ est 20% SO₃⁻;
- R₁ est de 25% à 30% SO₃⁻ dans les unités iduroniques et de 0 à 5% SO₃⁻ dans les unités glucuroniques;
- R est de 30% à 35% SO₃⁻ dans les unités glucuroniques and de 0 à 5% SO₃⁻ dans les unités iduroniques;
- la somme du pour-cent des SO₃⁻ dans R₁, unités glucuroniques and in R, unités iduroniques, est 5%;
R₁ et R n'étant pas simultanément SO₃⁻ et les deux étant hydrogène dans 30% à 40% des unités acide uronique; le degré de sulfatation étant de 2,5 à environ 2,7.

37. Un glycosaminoglycane selon la revendication 36, **caractérisé en ce que** ledit mélange de chaînes a un poids moléculaire moyen de 15.700.

38. Une composition pharmaceutique comprenant, en tant que principe actif, une quantité pharmacologiquement efficace d'un glycosaminoglycane selon une des revendications 22-37, sous forme d'un de ses sels pharmaceutiquement acceptables, et un excipient pharmaceutiquement acceptable.

39. Utilisation d'un glycosaminoglycane selon une des revendications 22-37 pour la préparation de compositions pharmaceutiques pour le contrôle de la coagulation dans un mammifère.

40. Utilisation d'un glycosaminoglycane selon une des revendications 22-37 pour la préparation de compositions pharmaceutiques pour la prévention ou le traitement de la thrombose dans un mammifère.

41. Utilisation selon une des revendications 39-40 **caractérisé en ce que** ladite composition pharmaceutique contient de 5 à 100 mg dudit glycosaminoglycane.
